# EUROPEAN PATENT APPLICATION

(11) **EP 3 309 550 A1**
(43) Date of publication of application: **18.04.2018**
(21) Application number: 16193587.9
(22) Date of filing: 12.10.2016
(51) Int. Cl.: G01N 33/543

(54) **METHOD FOR THE DETECTION OF APOLIPOPROTEIN E4**

(71) Applicant: sphingotec GmbH, 16761 Hennigsdorf (DE)
(72) Inventor: Bergmann, Andreas, 13465 Berlin (DE)
(74) Representative: Kilger, Ute

(57) **Abstract**

The present invention provides a method for the detection of Apolipoprotein E isotype 4 (ApoE4) or fragments thereof in a blood sample of a subject, whereby said method comprises the steps of contacting said sample with a solid phase, contacting said sample with at least one binder binding specifically to ApoE4 or a fragment thereof, thereby forming an ApoE4binder-complex, and detecting the ApoE4-binder-complex.

## Description

The present invention provides a simple and cost effective method to determine the Apolipoprotein E (ApoE) status of individuals. Those individuals include, but are not limited to, patients with cognitive impairment, dementia and/or Alzheimer's disease (AD).

Among the different susceptibility genes associated with the risk of late-onset Alzheimer's disease (LOAD) and other neurological conditions, ApoE has been identified as a strong genetic determinant (*Leduc et al. 2011).* The human APOE gene exists as three polymorphic alleles ε2, ε3 and ε4 which have a worldwide frequency of 8.4%, 77.9% and 13.7%. Apolipoprotein E (ApoE) is composed of 299 amino acids and is associated with lipoproteins in the plasma and the cerebrospinal fluid (*Leduc et al. 2011).* Differences between the three ApoE isoforms are limited to amino acids 112 and 158 (SEQ ID NO. 1), where either cysteine or arginine is present: ApoE2 (cys112, cys158), ApoE3 (cys112, arg158), and ApoE4 (arg112, arg158). Moreover, the risk of AD is strongly correlated with ApoE4 allele. Individuals with one ApoE4 allele have a five to six fold greater risk of developing AD, and individuals with two ApoE4 alleles have a more than 20 times greater risk for developing AD. Furthermore, the presence of one (heterozygous) or two copies (homozygous) of ApoE4 allele correlates with an earlier age of onset by about 10-20 years with regard to non-carriers in patients with late-onset disease (*Verghese et al. 2011, Weisgraber & Mahley 1996, Leduc et al. 2011*). Research suggests that individuals who meet the clinical criteria for mild cognitive impairment (MCI) who are also ApoE4 positive are more likely to progress to AD within a few years than individuals who are ApoE4 negative (*Aggarwal et al. 2005*). Therefore, determination of ApoE genotype can be used to identify MCI patients with a higher risk for progression from MCI to AD. ApoE4-carriers also respond differently to several kinds of AD treatment, e.g. to the acetylcholinesterase inhibitor tacrin, which raises the importance for the patients to know their genotype (*Cacabelos et al. 2012*).

Furthermore, the ApoE status can be used for the prediction of other diseases (individuals carrying the ApoE4 isoform have a higher risk of developing cardiovascular disease (CVD) and have also a higher risk for prostate cancer *(*US patent 5,945,289*)).* On the other hand, the ApoE4 genotype was shown to be protective against severe liver damage during a Hepatitis C infection, raises the viral load and fastens disease progression in HIV infected individuals, and elevates the risk of AD due to a Herpes simplex virus infection (WO03/060158 A2*).* Treatment of age-related macular degeneration benefits from the ApoE4 isoform as well (*Bakbak et al. 2015).*

The ApoE genotype as a risk marker for Alzheimer's disease was first described in Mayeux et al. 1998 and US 5,508,167. There is a variety of possibilities to detect the genetic status of ApoE in individuals by different genotyping methods.

It has been demonstrated that ApoE4 is much more susceptible to proteolysis than apoE3 and apoE2 and carboxyl-terminal truncated forms of apoE4 have been found in brains of AD patients and apoE4 transgenic mice. The apoE4 fragmentation has been proposed to be an early event in the pathogenesis of AD (Brecht W. J. et al. 2004. Neuron-specific apolipoprotein e4 proteolysis is associated with increased tau phosphorylation in brains of transgenic mice. PNAS 24, 2527-2534*).*

Several specific, possibly neurotoxic, carboxyl-terminal truncated apoE4 fragments have been found in the brain of AD patients:
the fragment ApoE4 1-165 with a molecular weight of 19 kDa (Huang Y. et al. 2001. Apolipoprotein E fragments present in Alzheimer's disease brains induce neurofibrillary tangle-like intracellular inclusions in neurons. 98, 8838-8843*),*
the fragment ApoE4 1-185 (Dafnis I. et al. 2010. An apolipoprotein E4 fragment can promote intracellular accumulation of amyloid peptide beta 42. 115, 873-884*),*
the fragments ApoE4 1-191 and ApoE4 1-260 (Tanaka M. 2006. Effect of carboxyl-terminal truncation on structure and lipid interaction of human apolipoprotein E4. Biochemistry. 45(13):4240-7*)*
the fragment ApoE4 1-272 (Chang S. et al. 2005. Proc Natl Acad Sci U S A. Lipid- and receptor-binding regions of apolipoprotein E4 fragments act in concert to cause mitochondrial dysfunction and neurotoxicity. 102(51):18694-9*)*
the fragments ApoE4 1-259, ApoE4 1-229 and ApoE4 1-202 (Dafvis I et al. 2015. Influence of Isoforms and Carboxyl-Terminal Truncations on the Capacity of Apolipoprotein E To Associate with and Activate Phospholipid Transfer Protein. Biochemistry. 54(38):5856-66*;* Vezeridis et al. 2011. Domains of apoE4 required for the biogenesis of apoE-containing HDL. Ann Med. 43(4):302-11*)*
the fragment ApoE4 1-231 (Chou C. Y. et al. 2006. Structural and functional variations in human apolipoprotein E3 and E4. J Biol Chem. 281(19):13333-44*)*
the fragments ApoE4 1-251 and ApoE4 1-266 (Dong L.M. 1994. Human apolipoprotein E. Role of arginine 61 in mediating the lipoprotein preferences of the E3 and E4 isoforms. J Biol Chem. 269(35):22358-65*)*

Moreover, N-terminal truncated ApoE4 fragments were investigated:
Fragment ApoE4 72-299 (Chou C.Y. et al. 2006. Structural and functional variations in human apolipoprotein E3 and E4. J Biol Chem. 281(19):13333-44*;* Chou C. Y. et al. 2005. Structural variation in human apolipoprotein E3 and E4: secondary structure, tertiary structure, and size distribution. Biophys J. 88(1):455-66*).*

Standard genotyping methods use isolated DNA from peripheral venous blood or mouth epithelial cells to analyze APOE gene polymorphism. The more frequent method has been traditionally PCR-RFLP (PCR-Restriction Fragment Length Polymorphism) analysis. However, it is a time consuming and error-prone method, due to possible incomplete restriction enzyme digestion. PCR plus sequencing or mass spectrometry are effective methods, but require expensive dedicated instrumentation. The ARMS-PCR (Amplification Refractory Mutation System-PCR) and SSP-PCR (Simple Sequence Specific Primer-PCR) methodologies require analysis by agarose gels, thus limiting the number of samples that can be examined at a time. The Real Time PCR detection by fluorescence melting curves is a simple and rapid method, but the formation of primer-dimers may complicate the melting curves interpretation. The Real Time PCR detection by fluorescence melting curves, the use of FRET (Fluorescent Resonance Energy Transfer) or TaqMan® are very effective, although costly methods. There are more and more rapid DNA based ApoE tests evolving (e.g. *Calero et al. 2009. Zhong et al. 2016),* but all of them rely on the aforementioned techniques.

There are also assays available based on the phenotype: e.g. isoelectric focusing or 2D gel electrophoresis, which are methods that are quite error prone due to posttranslational changes of ApoE isoforms. Another method is the immunochemical assay, which is described in the US patent US 6,027,896 and is based on the presence of reducible cysteine residues in the isoforms E2 and E3 but not E4. ApoE levels in bodily fluids can also be measured using commercially available immunoassays, usually sandwich type ELISAs. Those assays either measure all ApoE in bodily fluids, ApoE4 only or both.

It has been shown that ApoE from cerebrospinal fluid (CSF) binds to polypropylene, polystyrene, polyethylene and glass tubes (*Hesse et al. 2000, Holmquist 1982).* With these results Hesse et al. explained the differences in measurements of ApoE levels in CSF in different experiments and defined the binding of ApoE to those materials as a disturbing factor for immunoassays. Holmquist, 1982, describes the binding of extracted and purified serum ApoC and ApoE to plastic tubes and glass pipettes and underlines the error probability in ApoE immunoassays due to sample preparation. The fact that delipidated human serum apolipoproteins adsorb strongly to glass and plastic surfaces is an error source in double antibody immunoassays (e.g. Sandwich ELISA; *Holmquist 1982, Høgåsen et al. 1993).*

Høgåsen et al. 1993 showed that clusterin binds strongly to polystyrene microplates. This non-specific binding ability of clusterin is enhanced by the addition of Tween-20. They created a single antibody immunoassay, in which they pre-incubated plasma or serum samples in PBS-T (PBS with 0.2 % Tween-20) on a polystyrene microplate. Subsequently they detected and quantified immobilized clusterin using a clusterin specific antibody.

The present invention uses this ability of ApoE, in particular ApoE4 to strongly bind polymeric and glass surfaces and thus create a simple and cost-effective single antibody immunoassay method using blood, plasma or serum samples.

As described above, Hesse et al. 2000 shows that ApoE from the directly applied CSF binds to the polymeric or glass tubes and can be detected after elution (e.g. by SDS-elution and subsequent western blot analysis). However, in CSF there is an average total protein concentration of 0.15-0.45 mg/ ml. In contrast, plasma and serum have a 100- to 500-fold higher protein concentration compared to CSF, of 60-80 mg/ ml. It is common for immunoassays to saturate the binding surfaces with blocking proteins (e.g. bovine serum albumin) at concentrations of 0.5 to 2 mg/ ml to inhibit unspecific binding of sample proteins to the surfaces. ApoE concentrations in serum are in the range of 30 to 400 µg/ ml (*Bury et al. 1986*). Therefore, the estimated ApoE4 amount in plasma/ serum is only 0.02-0.5 % of the total plasma/ serum proteins and it would not be expected that one could measure a signal specific for ApoE4. Surprisingly, we are able to demonstrate, that ApoE4 from plasma, serum and even whole blood, binds specifically and quantifiably to the solid surfaces, even in the presence of a more than 200 to 5000-times higher total protein concentration in the sample.

Also surprisingly, ApoE4 binds specifically and quantifiably to the solid surfaces in the presence of blocking proteins (e.g. 0.5-2 mg/ ml bovine serum albumin (BSA)) in the reaction buffer. The presence of blocking proteins, such as BSA, can even enhance binding of ApoE4 to the solid phase.

A factor enhancing the specific binding of ApoE4 to polymeric and glass surfaces is the use of a detergent. Detergents are commonly used to prevent unspecific binding of sample proteins to the solid phase in immunoassays. Hesse et al. showed that ApoE binds to polystyrene, polypropylene, polyethylene and glass tubes in the presence of 0.2 % Tween-20 (*Hesse et al. 2000).*

Subject matter of the present invention is a method for (a) the identification of the ApoE4 status and/ or (b) the quantification of the ApoE4 levels in blood samples. The decision whether an individual is ApoE4 positive or ApoE4 negative (a) relies therein that the blood sample of a subject has an ApoE4 level above a certain threshold, which is defined by the internal cut-off control. The quantification of ApoE4 levels in blood samples (b) will be achieved by running internal standards with the samples and calculating the ApoE4-concentration with use of the standard curve.

### Detailed description of the embodiments of the present invention

Subject matter of the present invention is an (*in vitro*) method for the detection of Apolipoprotein E isotype 4 (ApoE4) or fragments thereof in a blood sample of a subject comprising the steps of:
a. contacting said sample with a solid phase,
   contacting said sample simultaneously or sequentially with at least one binder binding specifically to ApoE4, thereby forming an ApoE4-binder-complex, wherein said binder is an antibody or a functional antibody fragment or non-IgG protein scaffold, and
b. detecting the ApoE4-binder-complex, wherein said ApoE4-binder-complex is immobilized to said solid phase by means of ApoE4 or ApoE4 is immobilized to said solid phase and the binder to ApoE4.

In the context of the present invention, the ApoE4-binder-complex is immobilized via ApoE4 to the solid phase, in particular ApoE4 is immobilised to the solid phase and the binder is bound to ApoE4.

Subject matter of the present invention is further an assay based on the binding of an analyte (ApoE4 or fragments thereof) to a solid phase, in the presence of a detergent, and the detection of this immobilized analyte by an ApoE4 specific binder with a detectable label.

The ApoE4 specific binder can be labelled either directly or indirectly.

Subject matter of the present invention is also a method for the detection of ApoE4 or fragments thereof, wherein the blood sample of a subject is contacted with a solid phase thereby immobilizing ApoE4 to said solid phase.

The blood sample is selected from the group of whole blood, serum and plasma. Plasma is herein defined as EDTA-plasma, heparin-plasma and/ or citrate-plasma.

In the context of the present invention, the term "fragment of ApoE4" includes shorter splice variants of ApoE4 and truncated ApoE4 proteins or peptides or parts of ApoE4. (Rohn et al. 2013; Love et al. 2015).

These fragments comprise at least 6 amino acids in length with the proviso that the amino acid 158 (arginine, R) is encompassed within said fragment, whereby the sequence of the amino acid 158 refers to SEQ ID NO.1.

In a specific embodiment of the present invention, the fragments are selected from a group comprising the sequences of SEQ ID. NO. 1-15.

The term "subject" as used herein refers to a living human or or non-human organism, preferably a human subject, wherein the subject is healthy, apparently healthy, suffering from cognitive impairment, suffering from dementia, in particular Alzheimer's disease (AD).

In one embodiment of the method according to the present invention, the binding of ApoE4 or fragments thereof to the solid phase occurs without the interaction of a connecting molecule, which is able to bind/connect ApoE4 or fragments thereof to said solid phase.

In another embodiment of the method according to the present invention, at the moment of contacting the solid phase by the sample, said contacting occurs without the interaction of said connecting molecule.

In another embodiment of the method according to the present invention, the binding of ApoE4 or fragments thereof to the solid phase occurs in the presence of a detergent.

The detergent as used herein is selected from the group of anionic detergents (e.g. sodium dodecylsulfate (SDS)), cationic detergents (e.g. Cetyltrimethylammonium bromide (CTAB)), zwitterionic detergents (e.g. CHAPS) and non-ionic detergents (e.g. Tween-20, Triton X-100).

Examples of non-ionic detergents include epoxy-fatty acid esters, for example Tween-20 (polyoxyethylene-sorbitan monolaurate), Tween-80 (polyoxyethylene-sorbitan monooleate), Triton (alkyl-polyether-alcohol mixtures, e.g. Triton X-100, Triton X-114), Brij 35 and Brij 58 (polyoxyethylene lauryl ether), Nonidet P-40 (polyoxyethylene octylphenol ether), Lubrol PX (polyethylene oxide-alkyl ether adduct), Berol EMU 043 (C16, C18 fatty alcohol with 10 oxyethylene units); Deoxy-BIGCHAP, Digitonin, HECAMEG, N-D-Gluco-N-methylalkanamide (MEGA-8, 9, 10), n-Octyl-D-glucopyranoside, rac.-1-Oleoyl-glycerol, Pluronic® F-68, Sucrose monolaurate, Saponin from Quillaja Bark; and the like.

Zwitterionic detergents include, but are not limited to, CHAPS, CHAPSO, n-Octyl-β-D-glucopyranoside, Sulfobetaine SB 12 and Sulfobetaine SB 14.

Examples of cationic detergents include Benzethonium chloride, Cetylpyridinium chloride monohydrate, Cetyltrimethylammonium bromide (CTAB), Dodecyltrimethylammonium bromide (DTAB), and the like.

As an example of an anionic detergent, there is to be mentioned sodium dodecyl sulphate (SDS), Lithiumdodecylsulfat (LiDS), Sodium cholate, Sodium deoxycholate, N-Lauroylsarcosine sodium salt, 1-Decanesulfonic acid sodium salt, 1-Dodecanesulfonic acid sodium salt, 1-Heptanesulfonic acid sodium salt anhydrous, 1-Hexanesulfonic acid sodium salt anhydrous, 1-Nonanesulfonic acid sodium salt, 1-Octanesulfonic acid sodium salt, 1-Pentanesulfonic acid sodium salt anhydrous, and so on.

In one embodiment of the method according to the present invention, the detergent concentration in the reagent is preferably from 0.0001 to 10 % w/v and especially preferably from 0.01 to 1% w/v. A person skilled in the art knows that it may be considered to apply a specific concentration range for each specific detergent.

In a preferred embodiment of the method according to the present invention, Tween-20 is used in a concentration of 0.001-10 %, preferably 0.01-1 %, most preferably 0.2-0.5 %. In another embodiment of the method according to the present invention, Triton X-100 is used in a concentration of 0.001-0.5 %, preferably 0.01-0.2 %, most preferably 0.05-0.1 %.

In the present invention, detergents are necessary for the binding of ApoE4 or fragments thereof to the solid phase, but they do not act as connecting or binding molecules. Detergents have to be present in the reaction buffer, since there is no binding of ApoE4 to the solid phase in sample buffer without detergent (see example 4).

In a preferred embodiment of the method according to the present invention, the binder is specifically binding ApoE4 or fragments thereof.

In a further embodiment of the method according to the present invention, the binder is an antibody or a functional antibody fragment or non-IgG scaffold with a binding affinity constant of at least 10⁷ M⁻¹, preferred 10⁸ M⁻¹, preferred affinity constant is greater than 10⁹ M⁻¹, most preferred greater than 10¹⁰ M⁻¹.

In the context of the present invention, "binder molecules" are molecules which may be used to bind target molecules or molecules of interest, i.e. analytes (in the context of the present invention ApoE4 and fragments thereof), from a sample. Binder molecules must thus be shaped adequately, both spatially and in terms of surface features, such as surface charge, hydrophobicity, hydrophilicity, presence or absence of Lewis donors and/or acceptors, to specifically bind the target molecules or molecules of interest. Hereby, the binding may for instance be mediated by ionic, van-der-Waals, pi-pi, sigma-pi, hydrophobic or hydrogen bond interactions or a combination of two or more of the aforementioned interactions between the capture molecules and the target molecules or molecules of interest. In the context of the present invention, binder molecules may for instance be selected from the group comprising a nucleic acid molecule, a carbohydrate molecule, a PNA molecule, a protein, an antibody, a peptide or a glycoprotein. Preferably, the binder molecules are antibodies, including fragments thereof with sufficient affinity to a target or molecule of interest, and including recombinant antibodies or recombinant antibody fragments, as well as chemically and/or biochemically modified derivatives of said antibodies or fragments derived from the variant chain with a length of at least 12 amino acids thereof.

The ApoE4 antibody may have the formats known in the art. Examples are human antibodies, monoclonal antibodies, humanized antibodies, chimeric antibodies, CDR-grafted antibodies. In a preferred embodiment of the present invention, antibodies according to the present invention are recombinantly produced antibodies as e.g. IgG, a typical full-length immunoglobulin, or antibody fragments containing at least the F-variable domain of heavy and/or light chain as e.g. chemically coupled antibodies (fragment antigen binding) including but not limited to Fab-fragments including Fab minibodies, single chain Fab antibody, monovalent Fab antibody with epitope tags, e.g. Fab-V5Sx2; bivalent Fab (mini-antibody) dimerized with the CH3 domain; bivalent Fab or multivalent Fab, e.g. formed via multimerization with the aid of a heterologous domain, e.g. via dimerization of dHLX domains,e.g. Fab-dHLX-FSx2; F(ab')2-fragments, scFv-fragments, multimerized multivalent or/and multispecific scFv-fragments, bivalent and/or bispecific diabodies, BITE® (bispecific T-cell engager), trifunctional antibodies, polyvalent antibodies, e.g. from a different class than G; single-domain antibodies, e.g. nanobodies derived from camelid or fish immunoglobulins and numerous others.

In addition to anti-ApoE4 antibodies other biopolymer scaffolds are well known in the art to complex a target molecule and have been used for the generation of highly target specific biopolymers. Examples are aptamers, spiegelmers, anticalins and conotoxins.

Non-Ig scaffolds may be protein scaffolds and may be used as antibody mimics as they are capable to bind to ligands or antigens. Non-Ig scaffolds may be selected from the group comprising tetranectin-based non-Ig scaffolds (e.g. described in US 2010/0028995), fibronectin scaffolds (e.g. described in EP 1266 025), lipocalin-based scaffolds (e.g. described in WO 2011/154420), ubiquitin scaffolds (e.g. described in WO 2011/073214), transferring scaffolds (e.g. described in US 2004/0023334), protein A scaffolds (e.g. described in EP 2231860), ankyrin repeat based scaffolds (e.g. described in WO 2010/060748), microprotein (preferably microproteins forming a cystine knot) scaffolds (e.g. described in EP 2314308), Fyn SH3 domain based scaffolds (e.g. described in WO 2011/023685) EGFR-A-domain based scaffolds (e.g. described in WO 2005/040229) and Kunitz domain based scaffolds (e.g. described in EP 1941867). Non-Ig scaffolds may be peptide or oligonucleotide aptamers. Aptamers are usually created by selecting them from a large random sequence pool and are either short strands of oligonucleotides (DNA, RNA or X-NA; *Xu et al. 2010, Deng et al. 2014)* or short variable peptide domains attached to a protein scaffold (*Li et al. 2011*).

Binders that may be used for determining the level of ApoE4 or fragments thereof exhibit an affinity constant to ApoE4 of at least 10⁷ M⁻¹, preferred 10⁸ M⁻¹, preferred affinity constant is greater than 10⁹ M⁻¹, most preferred greater than 10¹⁰ M⁻¹. A person skilled in the art knows that it may be considered to compensate lower affinity by applying a higher dose of compounds and this measure would not lead out-of-the-scope of the invention.

In a more preferred embodiment of the method according to the present invention, the binder is labelled with a detectable label in order to be detected after having bound to ApoE4.

In a preferred embodiment of the present invention the label of the binder is selected from the group comprising chemiluminescent label, enzyme label, fluorescence label, radioiodine label. The amount of labelled antibody bound to the analyte is then measured by an appropriate method.

In another embodiment of the method according to the present invention, the label of the binder is selected from a group comprising rare earth cryptates or rare earth chelates in combination with fluorescence dye or chemiluminescence dye, in particular a dye of the cyanine type.

The preferred detection methods for determining the level of ApoE4 or fragments thereof comprise immunoassays in various formats such as for instance radioimmunoassay (RIA), chemiluminescence- and fluorescence-immunoassays, Enzyme-linked immunoassays (ELISA), Luminex-based bead arrays, protein microarray assays, and rapid test formats such as for instance immunochromatographic strip tests.

In the context of the present invention, fluorescence based assays comprise the use of dyes, which may for instance be selected from the group comprising FAM (5-or 6-carboxyfluorescein), VIC, NED, Fluorescein, Fluoresceinsothiocyanate (FITC), IRD-700/800, Cyanine dyes, auch as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

In the context of the present invention, chemiluminescence based assays comprise the use of dyes, based on the physical principles described for chemiluminescent materials in Kirk-Othmer, Encyclopedia of chemical technology. Preferred chemiluminescent dyes are acridiniumesters.

Chemiluminescent label may be acridinium ester label, steroid labels involving isoluminol labels and the like.

Enzyme labels may be lactate dehydrogenase (LDH), creatinine kinase (CPK), alkaline phosphatase, aspartate aminotransferase (AST), alanine aminotransferase (ALT), acid phosphatase, glucose-6-phosphate dehydrogenase and so on.

In another embodiment of the method according to the present invention, the solid phase is selected from the group of polymeric surface (e.g. polystyrene), glass surface, cellulose and cellulose derivatives (e.g. nitrocellulose, polyvinylidene fluoride, nylon). In another specific embodiment of the method according to the present invention, the solid phase is selected from the group of glass or polymeric surface, wherein polymeric surface comprises but are not limited to polystyrene, poly(divinylbenzene), styrene-acylate copolymer, styrene-butadiene copolymer, styrene-divinylbenzene copolymer, poly(styrene-oxyethylene), polymethyl methacrylate, polyurethane, polyglutaraldehyde, polyethylene imine, polyvinylpyrrolidone, N,N'-methylene bis-acrylamide, polyolefeins, polyethylene, polytetrafluoroethylene, polyethylene terephthalate, polypropylene, polyvinylchloride, polyvinylacetate, polyacrylonitrile, polysulfone, poly(ether sulfone), polydimethylsiloxane, pyrolized materials, block copolymers, and copolymers of the foregoing, silicones, or silica.

Any material is usable for such a surface/ solid phase so long as it serves to achieve the objectives of this invention. Preferable examples of surfaces include polymeric surfaces and glass-made surfaces. The group of polymeric surface comprises but is not limited to: polystyrene, poly(divinylbenzene), styrene-acylate copolymer, styrene-butadiene copolymer, styrene-divinylbenzene copolymer, poly(styrene-oxyethylene), polymethyl methacrylate, polyurethane, polyglutaraldehyde, polyethylene imine, polyvinylpyrrolidone, N,N'-methylene bis-acrylamide, polyolefeins, polyethylene, polytetrafluoroethylene, polyethylene terephthalate, polypropylene, polyvinylchloride, polyvinylacetate, polyacrylonitrile, polysulfone, poly(ether sulfone), polydimethylsiloxane, pyrolized materials, block copolymers, and copolymers of the foregoing, silicones, or silica, cellulose and cellulose derivatives (e.g. nitrocellulose, polyvinylidene fluoride, nylon). The preferred surface material is polystyrene.

Polystyrene is a hydrophobic polymer, which is readily derivatized by the manufacturer. Microtiter plates for EIAs are usually made from: 1) unmodified polystyrene (e.g. pureGrade BRANDplates®), 2) modified polystyrene which has an ionized and hydrophobic surface (e.g. lipoGrade BRANDplates®, Thermo Scientific Microlite 1+ plates), 3) polystyerene optimized for binding of IgG (hydrophilic and hydrophobic; e.g. Greiner High Binding plates, immuneGrade BRANDplates®), or 3) polystyrene with a hydrophilic or low binding surface (e.g. Thermo Scientific MultiSorp plates, hydroGrade BRANDplates®). Polymeric surfaces, including polystyrene microplates are modified by either physical bombardment by energetic particles (ions, electrons, fast neutrals and/ or radicals) and/ or by ultraviolet photons, or chemical reactions at or near the surface (e.g. treatment with acid or treatment with organosilanes) (*Hegemann et al. 2003,* WO 9203732*,* DE 2018523 A1). The preferred solid phase for the present invention is a high-binding polystyrene microplate.

In another embodiment of the present invention, the solid phase is unmodified (pure) or modified to be hydrophilic, hydrophobic or optimized for binding of IgG (high-binding: hydrophilic and hydrophobic) by physical bombardment by energetic particles (ions, electrons, fast neutrals and/ or radicals) and by ultraviolet photons, and/ or chemical reactions at or near the surface (e.g. treatment with acid or treatment with organosilanes).

In another specific embodiment of the method according to the present invention, the surface of the solid phase are used in suitable shapes, such as plates, multiwell plates, tubes or beads, sheets, films, particulates, magnetic or non-magnetic particles. In a preferred embodiment of the present invention the shape of the surface of the solid phase is a microtiter plate.

In a more specific embodiment of the method according to the present invention, the blood sample of the subject is contacted with an unsaturated solid phase. This means that the solid phase at the moment when it is contacted by the sample is not saturated/coated with blocking agents and/or binding molecules and/or stabilizing proteins and/or connecting molecules.

However, blocking agents or stabilizing proteins can support the existing binding of ApoE4 or fragments thereof to the solid phase and the binder when the sample has already contacted the solid phase, that means the stabilizing/blocking molecules/proteins can optionally be present during the term of the detection of the immobilized ApoE4-binder complex (during claimed step b.).

In the context of the present invention, the term "blocking agents" comprises but is not limited to protein solutions (e.g. of bovine serum albumin, albumin from human serum, milk proteins, casein, fish gelatin) or whole sera (e.g. calf serum).

In one embodiment of the method according to the present invention, the solid face is not covered with any binder or capture molecule. The definition of the term "binder", as used herein, is provided above.

In the context of the present invention, "capture molecules" are molecules which may be used to bind target molecules or molecules of interest, i.e. analytes (i.e. in the context of the present invention the peptide(s)), from a sample. Capture molecules must thus be shaped adequately, both spatially and in terms of surface features, such as surface charge, hydrophobicity, hydrophilicity, presence or absence of Lewis donors and/or acceptors, to specifically bind the target molecules or molecules of interest. Hereby, the binding may for instance be mediated by ionic, van-der-Waals, pi-pi, sigma-pi, hydrophobic or hydrogen bond interactions or a combination of two or more of the aforementioned interactions between the capture molecules and the target molecules or molecules of interest. In the context of the present invention, capture molecules may for instance be selected from the group comprising a nucleic acid molecule, a carbohydrate molecule, a PNA molecule, a protein, an antibody, a peptide or a glycoprotein. Preferably, the capture molecules are antibodies, including fragments thereof with sufficient affinity to a target or molecule of interest, and including recombinant antibodies or recombinant antibody fragments, as well as chemically and/or biochemically modified derivatives of said antibodies or fragments derived from the variant chain with a length of at least 12 amino acids thereof.

In another embodiment of the method according to the present invention, a stabilizing protein supports the existing binding of ApoE4 or fragments thereof in the blood sample to the solid phase, wherein said protein can optionally be added in the reaction buffer.

Reaction buffers of immunoassays usually contain stabilizing/ blocking proteins (e.g. bovine serum albumin, albumin from human sera, milk proteins, casein, gelatin, immune globulins). Those additional proteins prevent unspecific binding to the solid phase and stabilize the binding partners (binder and antigen), as well as the binder-antigen interaction. Surprisingly, the addition of such stabilizing/ blocking proteins to the reaction enhances the binding of ApoE4 or fragments thereof to the solid phase (see example 6).

In one further embodiment of the method according to the present invention, the stabilizing protein is selected from the group comprising bovine serum albumin (BSA), albumin from human serum, casein, milk proteins, immunoglobulins, stabilizing amino acids (e.g. Alanine, Arginine, Glycine, Isoleucine, Lysine, Proline, Serine), and the like. The preferred stabilizing protein is bovine serum albumin (BSA).

In another embodiment of the method according to the present invention, the concentration of BSA is in the range of 0-10 %, preferably 0.2-5%, most preferably 0.5-2.5 %.

In another specific embodiment of the method according to the present invention, the presence of other proteins than ApoE4 in the blood sample does not have any influence on the detection for ApoE4 or their fragments thereof.

In another embodiment of the method according to the present invention, contacting the blood sample of a subject with a solid phase, and contacting said sample with at least one binder binding specifically to ApoE4 or a fragment thereof occurs in a one-step or two-step procedure (see example 5).

In the context of the present invention, the term "one step procedure" means that the immobilization of ApoE4 or fragments thereof onto the solid phase and the binding of ApoE4 or fragments thereof by a labelled binder (e.g. antibody) is performed at the same time in the same vessel. During this kind of procedure ApoE4 alone and afterwards the specific binder, and/ or ApoE4-binder-complexes are immobilized on the solid phase.

As used herein, the term "two step procedure" means that the immobilization of ApoE4 or fragments thereof onto the solid phase and the binding of ApoE4 or fragments thereof by a specific binder takes place at stepwise time points, optional with a washing step between the incubation steps. For example, ApoE4 can first be immobilized onto the solid phase and subsequently, the ApoE4 specific labelled binder will bind immobilized ApoE4. Another possibility is that ApoE4 and the ApoE4 specific labelled binder are incubated in a first step to allow the formation of ApoE4-binder complexes, and in a second step ApoE4-binder-complexes are immobilized onto the solid phase.

In another specific embodiment of the method according to the present invention the ApoE4-binder-complex is detected qualitatively or quantitatively, wherein the qualitative detection of the ApoE4-binder-complex indicates the presence of ApoE4 or fragments thereof in said sample, wherein the quantitative detection of the ApoE4-binder-complex indicates the concentration of ApoE4 or fragments thereof in said sample.

In another embodiment of the method according to the present invention, the quantitative detection of the concentration of ApoE4 or fragments thereof indicates whether the subjects are homozygous or heterozygous for ApoE4 alleles.

In another specific embodiment of the method according to the present invention, the presence of the ApoE4-binder-complex is correlated with a risk of developing/incidence of AD.

In one specific embodiment of the method according to the present invention, the detection of ApoE4 is used for the determination of a risk to develop Alzheimer's disease (AD).

Subject matter of the present invention is also a method for predicting the risk of incidence of Alzheimer's Disease, as well as medical implications in all the before mentioned diseases and infections.

In one embodiment of the method according to the present invention, the detection of immobilized ApoE4 in the ApoE4-binder complex is used for a classification of a subject to be ApoE4 positive or ApoE4 negative, when the level of the detected ApoE4 in the ApoE4-binder complex is above a certain threshold, wherein said threshold is determined by a specific internal positive control.

In another embodiment of the method according to the present invention, a level of the ApoE4-binder-complex above a certain threshold is predictive for an enhanced risk of incidence of AD, wherein said threshold is defined by an internal cut-off control.

According to the method of the present invention, ApoE4-positive blood samples of subjects measure higher than the level of the specific internal cut-off control (= threshold). In a more preferred embodiment, the threshold is about 1.5 to 100 times lower than the level of ApoE4-positive samples, preferably 1.5 to 20 times lower, most preferred 1.5 to 10 times lower. ApoE4-negative samples measure lower than the internal cut-off control. In a more preferred embodiment, the threshold is about 10 to 10000 times higher than the level of ApoE4-negative samples, preferably 50 to 5000 times higher, most preferred 100 to 1000 times higher.

In the context of the present invention, an ApoE4 level above said threshold indicates the presence of ApoE4 in a patient and thus a high risk of developing/ incidence of AD. An ApoE4 level below said threshold indicates the absence of ApoE4 in a patient and thus a low risk of developing/ incidence of AD.

In a specific embodiment of the present invention for a qualitative detection of the ApoE4-binder complex, the level of ApoE4 or fragments thereof is measured with an immunoassay using antibodies or fragments of antibodies. ApoE4-positive and ApoE4-negative samples are divided by threshold defined by a specific internal cut-off control, wherein all ApoE4-negative samples show only background signal and all ApoE4-positive samples measure at least 1.5 times higher than the cut-off control (See examples 5, 6 and 7).

Another embodiment of the present invention is the evaluation of the measured units (i.e. fluorescence, luminescence, enzyme reaction etc.), which corresponds to the level of the detected ApoE4 levels, that can be quantitatively analyzed using ApoE4 calibrators with standard concentrations (see example 7). By means of a quantitative analysis the ApoE4-positive group according to the present invention can be further divided into two groups which correspond to subjects which are homozygous or heterozygous for ApoE4 alleles using a specific threshold level.

One embodiment of the present invention is the correlation of the presence of ApoE4/ the ApoE4-binder-complex with a risk of developing/ incidence of AD. The evaluation of the results indicates a low risk of developing/ incidence of AD for ApoE4 negative subjects and indicates a high risk of developing/ incidence of AD for ApoE4 positive subjects.

In one embodiment of the invention, the method is used for the risk stratification or stratification for application of therapeutic measures of a disease or condition associated with elevated ApoE4 levels.

In another embodiment of the invention, the disease or condition is selected from the group of cognitive impairment and neurodegenerative diseases including, dementia and/or Alzheimer's disease.

Subject-matter of the invention is also an assay which enables the performance of the method for detection of ApoE4 or fragments thereof.

In the context of the present invention, an assay comprises all the test components, which are necessary to perform the method of the present invention. However, as mentioned herein an "assay" is not only the box or the unit containing said test components, but rather the functional combination of all interacting components enabling the performance of the claimed method of the present invention.

Said assay of the present invention allows performing the method of the present invention also as a fully automated assay system, e.g. on a platform which is available on the market (Roche cobas®, Abbott Architect®, Siemens Centauer®, Brahms Kryptor®, Biomerieux Vidas®, Alere Triage®).

In a specific embodiment of the present invention, the assay enables the performance of the method for the detection of ApoE4 or fragments thereof in a blood sample of a subject, comprising the steps of:
a. contacting said sample with a solid phase,
   contacting said sample simultaneously or sequentially with at least one binder binding specifically to ApoE4, thereby forming an ApoE4-binder-complex, wherein said binder is an antibody or a functional antibody fragment or non-IgG protein scaffold, and
b. detecting the ApoE4-binder-complex, wherein said ApoE4-binder-complex is immobilized to said solid phase by means of ApoE4 or ApoE4 is immobilized to said solid phase and the binder to ApoE4.

In the context of the present invention, said assay enables the performance of the method of the present invention, wherein the ApoE4-binder-complex is immobilized via ApoE4 to the solid phase, in particular ApoE4 is immobilised to the solid phase and the binder is bound to ApoE4.

In one embodiment of the present invention said assay enables the performance of the method of the present invention, wherein the binding of ApoE4 to the solid phase occurs without the interaction of a connecting molecule, (which is able to bind/connect ApoE4 or fragments thereof to said solid phase).

In another embodiment of the present invention, said assay enables the performance of the method of the present invention, wherein at the moment of contacting the solid phase by the sample, said contacting occurs without the interaction of said connecting molecule.

In another embodiment of the present invention, said assay enables the performance of the method of the present invention, wherein the binding of ApoE4 to the solid phase occurs in the presence of a detergent.

In one embodiment of the present invention, said assay enables the performance of the method of the present invention, wherein the detergent concentration in the reagent is preferably from 0.0001 to 10 % w/v and especially preferably from 0.01 to 1% w/v. A person skilled in the art knows that it may be considered to apply a specific concentration range for each specific detergent.

In a preferred embodiment of the present invention, said assay enables the performance of the method of the present invention, wherein Tween-20 is used in a concentration of 0.001-10 %, preferably 0.01-1 %, most preferably 0.2-0.5 %. In another embodiment of the method according to the present invention, Triton X-100 is used in a concentration of 0.001-0.5 %, preferably 0.01-0.2 %, most preferably 0.05-0.1 %.

In a preferred embodiment of the present invention, said assay enables the performance of the method of the present invention, wherein the binder is specifically binding ApoE4 or fragments thereof.

In a further embodiment of the present invention, said assay enables the performance of the method of the present invention, wherein the binder is an antibody or a functional antibody fragment or non-IgG scaffold with a binding affinity constant of at least 10⁷ M⁻¹, preferred 10⁸ M⁻¹, preferred affinity constant is greater than 10⁹ M⁻¹, most preferred greater than 10¹⁰ M⁻¹.

In a more preferred embodiment of the present invention, said assay enables the performance of the method of the present invention, wherein the binder is labelled with a detectable label in order to be detected after having bound to ApoE4.

In a preferred embodiment of the present invention, said assay enables the performance of the method of the present invention, wherein the label of the binder is selected from the group comprising chemiluminescent label, enzyme label, fluorescence label, radioiodine label. The amount of labelled antibody bound to the analyte is then measured by an appropriate method.

In another embodiment of the present invention, said assay enables the performance of the method of the present invention, wherein the label of the binder is selected from a group comprising rare earth cryptates or rare earth chelates in combination with fluorescence dye or chemiluminescence dye, in particular a dye of the cyanine type.

In another embodiment of the present invention, said assay enables the performance of the method of the present invention, wherein the solid phase is selected from the group of polymeric surface (e.g. polystyrene), glass surface, cellulose and cellulose derivatives (e.g. nitrocellulose, polyvinylidene fluoride, nylon).

In another embodiment of the present invention, said assay enables the performance of the method of the present invention, wherein the solid phase is selected from the group of glass or polymeric surface, wherein polymeric surface comprises but are not limited to polystyrene, poly(divinylbenzene), styrene-acylate copolymer, styrene-butadiene copolymer, styrene-divinylbenzene copolymer, poly(styrene-oxyethylene), polymethyl methacrylate, polyurethane, polyglutaraldehyde, polyethylene imine, polyvinylpyrrolidone, N,N'-methylene bis-acrylamide, polyolefeins, polyethylene, polytetrafluoroethylene, polyethylene terephthalate, polypropylene, polyvinylchloride, polyvinylacetate, polyacrylonitrile, polysulfone, poly(ether sulfone), polydimethylsiloxane, pyrolized materials, block copolymers, and copolymers of the foregoing, silicones, or silica.

In another embodiment of the present invention, said assay enables the performance of the method of the present invention, wherein the solid phase is unmodified (pure) or modified to be hydrophilic, hydrophobic or optimized for binding of IgG (high-binding: hydrophilic and hydrophobic) by physical bombardment by energetic particles (ions, electrons, fast neutrals and/ or radicals) and by ultraviolet photons, and/ or chemical reactions at or near the surface (e.g. treatment with acid or treatment with organosilanes).

In another specific embodiment of the present invention, said assay enables the performance of the method of the present invention, wherein the surface of the solid phase are used in suitable shapes, such as plates, multiwell plates, tubes or beads, sheets, films, particulates, magnetic or non-magnetic particles. In a preferred embodiment of the present invention the shape of the surface of the solid phase is a microtiter plate.

In a more specific embodiment of the present invention, said assay enables the performance of the method of the present invention, wherein the blood sample of the subject is contacted with an unsaturated solid phase. This means that the solid phase at the moment when it is contacted by the sample is not saturated/coated with blocking agents and/or binding molecules and/or stabilizing proteins and/or connecting molecules.

However, blocking agents or stabilizing proteins can support the existing binding of ApoE4 to the solid phase and the binder when the sample has already contacted the solid phase, that means the stabilizing/blocking molecules/proteins can optionally be present during the term of the detection of the immobilized ApoE4-binder complex (during step b.)

In one embodiment of the present invention, said assay enables the performance of the method of the present invention, wherein the solid face is not covered with any binder or capture molecule. The definition of the term "binder", as used herein, is provided above.

In another embodiment of the present invention, said assay enables the performance of the method of the present invention, wherein a stabilizing protein supports the existing binding of ApoE4 in the blood sample to the solid phase, wherein said protein can optionally be added in the reaction buffer.

In one further embodiment of the present invention, said assay enables the performance of the method of the present invention, wherein the stabilizing protein is selected from the group comprising bovine serum albumin (BSA), albumin from human serum, casein, milk proteins, immunoglobulins, stabilizing amino acids (e.g. Alanine. Arginine, Glycine, Isoleucine, Lysine, Proline, Serine), and the like. The preferred stabilizing protein is bovine serum albumin (BSA).

In another embodiment of the present invention, said assay enables the performance of the method of the present invention, wherein the concentration of BSA is in the range of 0-10 %, preferably 0.2-5%, most preferably 0.5-2.5 %.

In another specific embodiment of the present invention, said assay enables the performance of the method of the present invention, wherein the presence of other proteins than ApoE4 in the blood sample does not have any influence on the detection for ApoE4 or their fragments thereof.

In another embodiment of the present invention, said assay enables the performance of the method of the present invention, wherein contacting the blood sample of a subject with a solid phase, and contacting said sample with at least one binder binding specifically to ApoE4 or a fragment thereof occurs in a one-step or two-step procedure (see example 5).

In another specific embodiment of the present invention, said assay enables the performance of the method of the present invention, wherein the method for the detection of ApoE4 or fragments thereof, the ApoE4-binder-complex is detected qualitatively or quantitatively, wherein the qualitative detection of the ApoE4-binder-complex indicates the presence of ApoE4 in said sample, wherein the quantitative detection of the ApoE4-binder-complex indicates the concentration of ApoE4 in said sample.

In another embodiment of the present invention, said assay enables the performance of the method of the present invention, wherein the quantitative detection of the concentration of ApoE4 indicates whether the subjects are homozygous or heterozygous for ApoE4 alleles.

In another specific embodiment of the present invention, said assay enables the performance of the method of the present invention, wherein the presence of the ApoE4-binder-complex is correlated with a risk of developing/incidence of AD.

In one specific embodiment of the present invention, said assay enables the performance of the method of the present invention, wherein the detection of ApoE4 or fragments thereof is used for the determination of a risk to develop Alzheimer's disease (AD).

In another specific embodiment of the present invention, said assay enables the performance of the method of the present invention, wherein the detection of ApoE4 or fragments thereof is used for predicting the risk of incidence of Alzheimer's Disease, as well as medical implications in all the before mentioned diseases and infections.

In another embodiment of the present invention, said assay enables the performance of the method of the present invention, wherein the detection of immobilized ApoE4 in the ApoE4-binder complex is used for a classification of a subject to be ApoE4 positive or ApoE4 negative, when the level of the detected ApoE4 in the ApoE4-binder complex is above a certain threshold, wherein said threshold is determined by a specific internal positive control.

In another embodiment of the present invention, said assay enables the performance of the method of the present invention, wherein a level of the ApoE4-binder-complex above a certain threshold is predictive for an enhanced risk of incidence of AD, wherein said threshold is defined by an internal cut-off control.

In a specific embodiment of the present invention, an assay is used for determining the level of ApoE4 and fragments thereof having at least 6 amino acids, wherein the assay sensitivity of said assay is able to detect/quantify ApoE4 and fragments thereof in a blood sample of a subjects and is < 1 ng/mL, preferably < 5 ng/mL, more preferably < 10 ng/mL and even more preferred < 25 ng/mL.

Subject-matter of the invention is also an assay which enables the performance of the method of the present invention , wherein the level of ApoE4 or fragments thereof is measured using an immunoassay comprising one or more capture probes directed against one or more epitopes of ApoE4 or fragments thereof.

In one embodiment of the present invention, said immunoassay is selected from a group comprising radioimmunoassays (RIA), homogeneous enzyme-multiplied immunoassays (EMIT), enzyme linked immuno adsorbent assays (ELISA), apoenzyme reactivation immunoassay (ARIS), dipstick immunoassays and immuno-chromatography assays.

In another embodiment of the present invention, such an assay may be a so-called POC-test (point-of-care), that is a test technology, which allows performing the test within less than 1 hour near the patient without the requirement of a fully automated assay system. One example for this technology is the immunochromatographic test technology.

In another embodiment of the present invention, such an assay is a single antibody immunoassay using any kind of detection technology including but not restricted to enzyme label, chemiluminescence label, electrochemiluminescence label, preferably a fully automated assay. Examples of automated or fully automated assay comprise assays that may be used for one of the following systems: Roche Elecsys®, Abbott Architect®, Siemens Centauer®, Brahms Kryptor®, Biomerieux Vidas®, Alere Triage®.

A further subject-matter of the present invention is an assay which enables the performance of the method of the present invention, comprising the test components:
a. one binder directed against an epitope comprised in the sequence of ApoE4 or fragments thereof, wherein the binder is labelled with a detectable label,
b. a solid phase (e.g. plate, tube, beads, sheet),
c. a detergent containing reaction buffer.

Another embodiment of the present invention is an assay which enables the performance of the method of the present invention, comprising:
a. one binder directed against an epitope comprised in the sequence of ApoE4 or fragments thereof, wherein the binder is labelled with a detectable label,
b. a polymeric or glass solid phase, preferentially a high-binding polystyrene microplate,
c. a reagent that contains detergent, preferably 0.2 % Tween-20.

The assay may additionally comprise controls:
▪ At least one of the following controls is necessary: negative control, cut-off control and positive control,
▪ controls may be samples with known ApoE4 concentration (native, purified or recombinant).

Another embodiment of the present invention is an assay, which enables the performance of the method of the present invention for the quantification of ApoE4 or fragments thereof in a sample of a subject comprising:
▪ one binder directed against an epitope comprised in the sequence of ApoE4 or fragments thereof, the binder is labelled with a detectable label,
▪ a polymeric or glass solid phase, preferentially a high-binding polystyrene microplate,
▪ a reagent that contains detergent, preferably 0.2 % Tween-20.

The assay may additionally comprise one or more calibrators:
▪ A calibrator may be samples with known ApoE4 concentration (native, purified or recombinant).

Another embodiment of the present invention is an assay, which enables the method for detection of ApoE4 or fragments thereof, consisting of:
▪ one binder directed against an epitope comprised in the sequence of ApoE4 or fragments thereof having at least 6 amino acids, wherein the binder is labelled with a detectable label,
▪ a polymeric or glass solid phase, preferentially a high-binding polystyrene microplate,
▪ a reagent that contains detergent, preferentially 0.2 % Tween-20,
▪ controls and/ or calibrators,
▪ washing buffer,
▪ measuring reagents.

Another subject-matter of the present invention is a kit for detecting ApoE4 or fragments thereof in a blood sample of a subject.

As mentioned herein, a "kit" is a box comprising a combination of materials, which are necessary to perform the method of the present invention, in particular the detection of ApoE4 and fragments thereof in a blood sample of a subject.

A further subject-matter of the present invention is a kit for detecting ApoE4 and fragments thereof in a blood sample of a subject comprising:
a. one binder directed against an epitope comprised in the sequence of ApoE4 or fragments thereof, wherein the binder is labelled with a detectable label,
b. a solid phase (e.g. plate, tube, beads, sheet),
c. a detergent containing reaction buffer.

Another embodiment of the present invention is a kit for detecting ApoE4 and fragments thereof in a blood sample of a subject comprising:
▪ one binder directed against an epitope comprised in the sequence of ApoE4 or fragments thereof, the binder is labelled with a detectable label,
▪ a polymeric or glass solid phase, preferentially a high-binding polystyrene microplate,
▪ a reagent that contains detergent, preferentially 0.2 % Tween-20.

The kit may additionally comprise controls:
▪ At least one of the following controls is necessary: negative control, cut-off control and positive control,
▪ controls may be samples with known ApoE4 concentration (native, purified or recombinant).

Another embodiment of the present invention is a kit for the quantification of ApoE4 or fragments thereof in a sample of a subject comprising:
▪ one binder directed against an epitope comprised in the sequence of ApoE4 or fragments thereof, the binder is labelled with a detectable label,
▪ a polymeric or glass solid phase, preferentially a high-binding polystyrene microplate,
▪ a reagent that contains detergent, preferentially 0.2 % Tween-20.

The kit may additionally comprise one or more calibrators:
▪ A calibrator may be samples with known ApoE4 concentration (native, purified or recombinant).

Another embodiment of the present invention is a kit for detecting ApoE4 or fragments thereof in a blood sample of a subject consisting of:
▪ one binder directed against an epitope comprised in the sequence of ApoE4 or fragments thereof having at least 6 amino acids, wherein the binder is labelled with a detectable label,
▪ a polymeric or glass solid phase, preferentially a high-binding polystyrene microplate,
▪ a reagent that contains detergent, preferentially 0.2 % Tween-20,
▪ controls and/ or calibrators,
▪ washing buffer,
▪ measuring reagents.

Another embodiment of the present invention is the use of a binder to detect ApoE4 or fragments thereof in a blood sample of a subject comprising the steps of:
a. contacting said sample with a solid phase,
   contacting said sample simultaneously or sequentially with at least one binder binding specifically to ApoE4, thereby forming an ApoE4-binder-complex, wherein said binder is an antibody or a functional antibody fragment or non-IgG protein scaffold, and
b. detecting the ApoE4-binder-complex, wherein said ApoE4-binder-complex is immobilized to said solid phase by means of ApoE4 or ApoE4 is immobilized to said solid phase and the binder to ApoE4.

In the context of the present invention claiming the use of a binder in the method of the present invention is synonymous with claiming the method of the present invention including all aforementioned embodiments.

The following embodiments are also subject of the present invention:
1. *(In vitro)* Method for the detection of Apolipoprotein E isotype 4 (ApoE4) or fragments thereof in a blood sample of a subject comprising the steps of:
   a. contacting said sample with a solid phase,
      contacting said sample simultaneously or sequentially with at least one binder binding specifically to ApoE4, thereby forming an ApoE4-binder-complex, wherein said binder is an antibody or a functional antibody fragment or non-IgG protein scaffold, and
   b. detecting the ApoE4-binder-complex, wherein said ApoE4-binder-complex is immobilized to said solid phase by means of ApoE4.
2. The method according to embodiment 1, wherein ApoE4 or fragments thereof is immobilized to said solid phase and the binder is bound to ApoE4.
3. The method according to embodiments 1 to 2, wherein the fragments of ApoE4 comprise at least 6 amino acids in length with the proviso that the amino acid 158 (Arginine) is encompassed within said fragment, whereby the sequence of the amino acid 158 refers to Seq ID NO.1.
4. The method according to embodiments 1 to 3, wherein the binding of ApoE4 or the ApoE4-binder-complex to said solid phase occurs without the interaction of a connecting molecule, which is able to bind ApoE4 or fragments thereof to said solid phase.
5. The method according to embodiments 1 to 4, wherein at the moment of contacting the solid phase by the sample, said contacting occurs without the interaction of said connecting molecule.
6. The method according to embodiments 1 to 5, wherein binding of ApoE4 or the ApoE4-binder-complex to said solid phase occurs in the presence of a detergent.
7. The method according to embodiments 1 to 6, wherein the ApoE4-binder-complex is detected qualitatively or quantitatively.
8. The method according to embodiments 1 to 7, wherein a quantitative detection of the ApoE4-binder-complex indicates the concentration of ApoE4 in said sample, wherein the concentration provides an indication whether the subjects are homozygous or heterozygous for ApoE4 alleles.
9. The method according to embodiments 1 to 8, wherein the binder is an antibody or a functional antibody fragment or non-IgG protein scaffold with a binding affinity constant of at least 10⁷ M⁻¹, preferred 10⁸ M⁻¹, preferred affinity constant is greater than 10⁹ M⁻¹, most preferred greater than 10¹⁰ M⁻¹.
10. The method according to embodiments 1 to 9, wherein the binder is labelled with a label in order to be detected, wherein said label is selected from the group comprising chemiluminescent label, enzyme label, fluorescence label or radioiodine label.
11. The method according to embodiments 1 to 10, wherein the detection of ApoE4 is used for a classification of a subject to be ApoE4 positive or ApoE4 negative, when the level of the detected ApoE4 is above a certain threshold, wherein said threshold is determined by a specific internal positive control.
12. The method according to embodiments 1 to 11, wherein the solid phase is selected from the group of polymeric surface (e.g. polystyrene), glass surface, cellulose and cellulose derivatives (e.g. nitrocellulose, polyvinylidene fluoride, nylon).
13. The method according to embodiments 1 to 12, wherein the solid phase is selected from the group of glass or polymeric surface, wherein polymeric surface comprises but is not limited to polystyrene, poly(divinylbenzene), styrene-acylate copolymer, styrene-butadiene copolymer, styrene-divinylbenzene copolymer, poly(styrene-oxyethylene), polymethyl methacrylate, polyurethane, polyglutaraldehyde, polyethylene imine, polyvinylpyrrolidone, N,N'-methylene bis-acrylamide, polyolefeins, polyethylene, polytetrafluoroethylene, polyethylene terephthalate, polypropylene, polyvinylchloride, polyvinylacetate, polyacrylonitrile, polysulfone, poly(ether sulfone), polydimethylsiloxane, pyrolized materials, block copolymers, and copolymers of the foregoing, silicones, or silica.
14. The method according to embodiments 1 to 13, wherein said blood sample is contacted with an unsaturated solid phase.
15. The method according to embodiments 1 to 14, wherein the solid face is not covered with any binder or capture molecule.
16. The method according to embodiments 1 to 15, wherein an optional stabilizing protein in the reaction buffer stabilizes the binding of ApoE4 and fragments thereof in the blood sample to the solid phase by blocking unspecific binding sites and interacting with the conformation of ApoE4, wherein the stabilizing protein is selected from the group comprising bovine serum albumin (BSA), albumin from human serum, casein, milk proteins, immunoglobulins and stabilizing amino acids (e.g. Alanine, Arginine, Glycine, Isoleucine, Lysine, Proline, Serine), preferably the stabilizing protein is bovine serum albumin (BSA).
17. The method according to embodiments 1-16, wherein the concentration of BSA as stabilizing protein is in the range of 0-10 %, preferably 0.2-5%, most preferably 0.5-2.5 %.
18. The method according to embodiments 1-17, wherein the detergent is selected from the group of anionic detergents (e.g. sodium dodecylsulfate (SDS)), cationic detergents (e.g. Cetyltrimethylammonium bromide (CTAB)), zwitterionic detergents (e.g. CHAPS) and/ or non-ionic detergents (e.g. Tween-20, Triton X-100).
19. The method according to embodiments 1-18, wherein the Tween-20 concentration is selected from a range of 0.001-10%, preferably 0.01-1 %, most preferably 0.2-0.5 %.
20. The method according to embodiments 1-19, wherein the Triton X-100 concentration is selected from a range of 0.001-0.5 %, preferably 0.01-0.2 %, most preferably 0.05-0.1 %.
21. The method according to any of the preceding embodiments, wherein the blood sample is selected from the group of whole blood, serum and citrate plasma, heparin plasma, EDTA-plasma.
22. The method according to any of the preceding embodiments, wherein the subject is selected from the group of living human or human-organism, preferably a human subject, wherein the subject is healthy, apparently healthy, diseased with cognitive impairment dementia, in particular AD.
23. A Kit comprising
   a. one binder directed against an epitope comprised in the sequence of ApoE4, wherein the binder is labelled with a detectable label,
   b. a solid phase,
   c. a detergent containing reaction buffer.
24. Use of a kit according to any of the preceding embodiments for the *in vitro* detection and/or quantification of ApoE4 or fragments thereof in a blood sample of a subject.
25. Use of the method according to any of the preceding embodiments for the risk stratification, or stratification for application of therapeutic measures of a disease or condition associated with elevated ApoE4 levels.
26. Use of the method according to any of the preceding embodiments, wherein the disease or condition is selected from the group of cognitive impairment and neurodegenerative diseases including dementia and/or Alzheimer's disease (AD).
27. A method according to any of the preceding embodiments for detecting the risk to develop AD, wherein an elevated level of ApoE4 above a certain threshold is predictive for an enhanced risk of developing AD.
28. The method according to any of the preceding embodiments, wherein said presence of ApoE4 or the ApoE4-binder-complex is correlated with a risk of developing/incidence of AD.

### Examples

### 1. Example 1

Generation of Antibodies and determination of their affinity constants.

### - Peptides/ conjugates for immunization:

A specific ApoE4 peptide for immunization and a ApoE2/3 specific control peptide were synthesized, see table 1, (JPT Technologies, Berlin, Germany). The ApoE4 peptide was synthesized with an additional N-terminal cystein residue for conjugation of the peptide to Bovine Serum Albumin (BSA). The peptide was covalently linked to BSA using Sulfolink-coupling gel (Perbio-science, Bonn, Germany). The coupling procedure was performed according to the manual of Perbio.

**Table 1:**

| **Peptides** | **ApoE Region** | **Description** | **Affinity constant [M⁻¹]** |
|---|---|---|---|
| Cys-EDVRGRLVQYR | 109-119 ApoE4 | Immunogen | 2.1 x 10⁹ |
| EDVCGRLVQYR | 109-119 ApoE2/3 | Control peptide | - |

### - Immunization of mice, immune cell fusion and screening:

A Balb/c mouse was immunized with 100 µg Peptide-BSA-Conjugate at day 0 and 14 (emulsified in 100 µl complete Freund's adjuvant) and 50 µg at day 21 and 28 (in 100 µl incomplete Freund's adjuvant). Three days before the fusion experiment was performed, the animal received 50 µg of the conjugate dissolved in 100 µl saline, given as one intraperitoneal and one intra venous injection.

Splenocytes from the immunized mouse and cells of the myeloma cell line SP2/0 were fused with 1 ml 50% polyethylene glycol for 30 s at 37°C. After washing, the cells were seeded in 96-well cell culture plates. Hybrid clones were selected by growing in HAT medium [RPMI 1640 culture medium supplemented with 20% fetal calf serum and HAT-Supplement]. After two weeks the HAT medium is replaced with HT Medium for three passages followed by returning to the normal cell culture medium.

The cell culture supernatants were primary screened for antigen specific IgG antibodies three weeks after fusion. The positive tested microcultures were transferred into 24-well plates for propagation. After retesting the selected cultures were cloned and recloned using the limiting-dilution technique and the isotypes were determined (*Lane et al. 1985, Ziegler et al. 1996).*

### - Monoclonal antibody production

Antibodies were produced via standard antibody production methods (*Marx et al. 1997*) and purified via Protein A. The antibody purities were > 95% based on SDS gel electrophoresis analysis.

### - Affinity Constants

To determine the affinity of the antibody to ApoE4, the kinetics of binding of recombinant ApoE4 to immobilized antibody was determined by means of label-free surface plasmon resonance using a Biacore 2000 system (GE Healthcare Europe GmbH, Freiburg, Germany). Reversible immobilization of the antibodies was performed using an anti-mouse Fc antibody covalently coupled in high density to a CM5 sensor surface according to the manufacturer's instructions (mouse antibody capture kit; GE Healthcare).

### - Labelling procedure (tracer)

100 µg (100 µl) of antibody (1 mg/ ml in PBS, pH 7.4,) was mixed with 10 µl Akridinium NHS-ester (1 mg/ml in acetonitrile, InVent GmbH, Germany; EP 0 353 971) and incubated for 20 min at room temperature. Labelled antibody was purified by Gel-filtration HPLC on Bio-Sil® SEC 400-5 (Bio-Rad Laboratories, Inc., USA). The purified labelled antibody was diluted in (300 mmol/L potassium phosphate, 100 mmol/L NaCl, 10 mmol/L Na-EDTA, 5 g/L Bovine Serum Albumin, pH 7.0). The final concentration was approx. 800.000 relative light units (RLU) of labelled compound (approx. 20 ng labelled antibody) per 200 µL. Akridiniumester chemiluminescence was measured by using an AutoLumat LB 953 (Berthold Technologies GmbH & Co. KG).

### 2. Example 2

Measurement of plasma samples with known genotype. Using plasma and whole blood matches one could analyze the ApoE status genetically by genotyping and measure ApoE4-levels in the matched plasma with the ApoE4 assay of the present invention.

### 2.1 Methods

### - Genotyping

Genetic analysis from 180 whole blood samples was performed by Medigenomix, Ebersberg, Deutschland). DNA from the samples was isolated and the ApoE gene sequenced using the Sanger sequencing technique (*Sanger & Coulson 1975*).

### - ApoE4 Assay procedure

ApoE4 single-antibody assay was performed in a one-step procedure. Plasma samples were directly pipetted into wells of a 96-well microtiter plate (Greiner, Lumitrac600 high-binding, polystyrene; 20 µl sample per well) in duplicates. A low ApoE4 cut-off control was added to each microplate (recombinant ApoE4 [4 µg/ ml] in ApoE4-negative human plasma, 20 µl per well, in duplicate). Wells were filled with 200 µl of reaction buffer (PBS with 0.2 % Tween-20, 1 % BSA, 0.1 % bovine IgGs, 0.1 % sheep IgGs and 0.02 % mouse IgGs, pH 7.4) with acridiniumester-labelled anti-ApoE4-antibody (tracer). The microplates were incubated for 1 h at room temperature. Unbound tracer was removed by washing 4 times with washing solution (20 mM PBS, pH 7.4, 0.1 % Triton X-100). Luminescence (in relative light units (RLU)) was measured using a MTP luminometer (Centro LB960, Berthold Technologies GmbH, Bad Wildbad).

### 2.2 Results

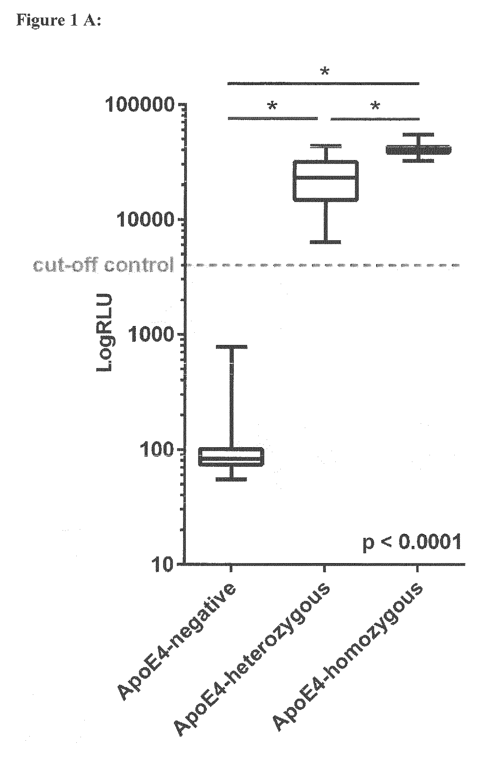

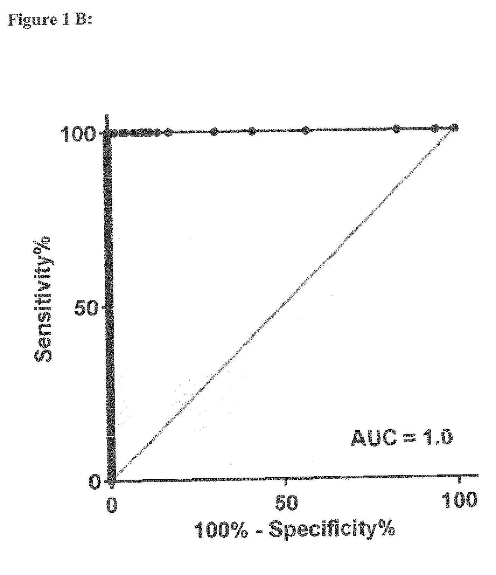

Figure 1 a shows results of the ApoE4 Assay differentiated by genotype. With the help of the cut-off control one can easily divide between ApoE4 positive and ApoE4 negative samples from the RLU-values. All ApoE4-negative samples have RLU values below 3000 RLU and all ApoE4-positive samples have RLU values above 3000 RLU. The negative samples are basically only showing the background signal of the immune assay, and no signal for ApoE4. Figure 1b shows the corresponding ROC curve. With an area under the curve (AUC) of 1, the assay is exactly differentiate between ApoE4-positive and -negative samples.

### 3. Example 3

Analysis of ApoE4 binding to different polymeric and glass surfaces with/ without detergent.

### 3.1 Methods

### - Pre-incubation of plasma samples on polymeric or glass surfaces:

Each ApoE4 positive plasma sample was split into 120 µl aliquots, which were incubated in tubes of different material (polystyrene (PS), polypropylene (PP), polyethylene terephthalate (PET) and glass), two tubes per material. To one half of the aliquots 0.1 % Triton X-100 was added before incubation, the other half was treated with an equal volume of PBS. All tubes were incubated at room temperature overnight.

### - ApoE4 single antibody assay

20 µl of each preparation, as well as the not pre-incubated control, were pipetted onto a Greiner high-binding microtiterplate per well. The ApoE4 assay was then performed as described in example 2. The measured ApoE4 concentration was calculated relatively to the concentration of the respective control sample (defined as 100 %).

### 3.2 Results

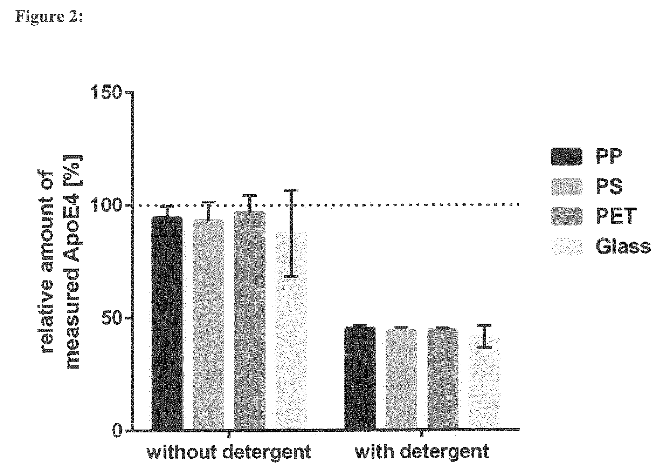

Detergent treated samples show a strong decrease in ApoE4 level after pre-incubation in polymeric or glass tubes, about 50 % loss (see figure 2). There is no difference between the type of surface the samples were pre-incubated on. There is a slight loss of ApoE4-signal after pre-incubation of the not-detergent-treated samples, but not as significant as with detergent. Detergent enhances the binding of ApoE4 to polymeric or glass surfaces.

### 4. Example 4

Analysis whether detergents act as linking molecules between ApoE4 and the solid phase.

### 4.1 Methods

### - Coating of microtiterplates (MTPs)

Three Greiner high-binding microtiterplates were filled with 300 µl per well of either ddH₂O, 0.05 % Triton X-100 solution in ddH₂O or 0.4 % Triton X-100 solution in ddH₂O (one plate each). The plates were incubated over night at room temperature. Afterwards MTPs were washed three times with 1xPBS and directly used for the ApoE4 one step single antibody assay.

### - ApoE4 single antibody assay

20 µl of plasma of ApoE4 positive and negative samples, as well as the cut-off control (4 µg/ ml recombinant ApoE4 in human plasma), were pipetted onto the pre-treated Greiner high-binding microtiterplates per well. One half of the wells was filled with 200 µl of tracer buffer (PBS with 1 % BSA, 0.1 % bovine IgGs, 0.1 % sheep IgGs and 0.02 % mouse IgGs, pH 7.4 + acridiniumester-labelled anti-ApoE4-antibody) containing 0.05 % Triton X-100, and the other half was filled with the same tracer buffer without detergent (0 % Triton X-100). The microplates were incubated for 1 h at room temperature. Unbound tracer was removed by washing 4 times with washing solution (20 mM PBS, pH 7.4, 0.1 % Triton X-100). Luminescence (in relative light units (RLU)) was measured using a MTP luminometer (Centro LB960, Berthold Technologies GmbH, Bad Wildbad).

### 4.2 Results

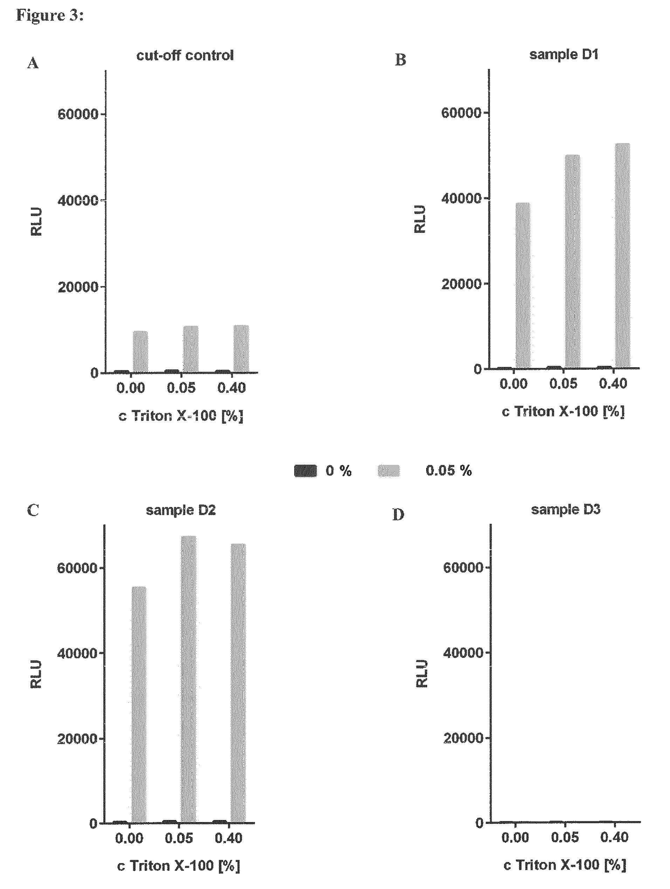

If detergents would act as a linking molecule between ApoE4 and the solid phase, there should be a strong signal when the ApoE4 assay is performed on detergent treated plates without any detergent in the assay buffer, possibly even concentration dependent. This is not the case. All samples, as well as the cut-off control, show no signal on any pre-treated plate when the assay is performed in assay buffer without any detergent (see figure 3). The signals of both, ApoE4-positive and ApoE4-negative samples are at baseline on the same level and can thus not be differentiated from each other. In contrast, when the assay is performed with detergent in the assay buffer, there is a significant signal in ApoE4-positive samples and the cut-off control. This signal is also independent from the amount of Triton X-100 coupled to the MTP.

Detergent is a necessary component of the buffer in the ApoE4 assay. Coating of detergent onto the MTP and adding sample and detergent-free buffer does not result in a significant signal. This shows that detergent is rather needed in its mobile form and not immobilized as possibly linking molecule.

### 5. Example 5

Comparison of one-step and two-step assay procedure.

### 5.1 Methods

### - One-Step Assay procedure

The ApoE4 single antibody assay was performed as described in example 2. In the one-step procedure samples and tracer antibody were incubated together for 1 hour at room temperature. Luminescence (in relative light units (RLU)) was measured using a MTP luminometer (Centro LB960, Berthold Technologies GmbH, Bad Wildbad). Added to each microplate, there was a low ApoE4-cut off control (recombinant ApoE4 [4 µg/ ml] in ApoE4-negative human plasma; 20 µl per well, in duplicate).

### - Two-Step Assay procedure

In the two-step procedure, plasma samples were directly pipetted into wells of a 96-well microtiter plate (Greiner, Lumitrac600 high-binding, polystyrene; 20 µl sample per well) in duplicates. Added to each microplate, there was a low ApoE4-cut off control (recombinant ApoE4 [4 µg/ ml] in ApoE4-negative human plasma; 20 µl per well, in duplicate). Wells were filled with 200 µl of reaction buffer (PBS with 0.2 % Tween-20, 1 % BSA, 0.1 % bovine IgGs, 0.1 % sheep IgGs and 0.02 % mouse IgGs, pH 7.4). The microplates were incubated over night at room temperature (= step 1). Afterwards, microplates were washed 4 times with washing solution (20 mM PBS, pH 7.4, 0.1 % Triton X-100) and 200 µl of reaction buffer with acridiniumester-labelled antiApoE4-antibody were filled into the wells. The microplates were incubated for 2 hours at room temperature (= step 2), and washed again 4 times with washing solution. Luminescence (in relative light units (RLU)) was measured using a MTP luminometer (Centro LB960, Berthold Technologies GmbH, Bad Wildbad).

In order to compare the two assay procedure variants more directly, the RLU values were transformed into the relative units "folds of cut-off control".

### 5.2 Results

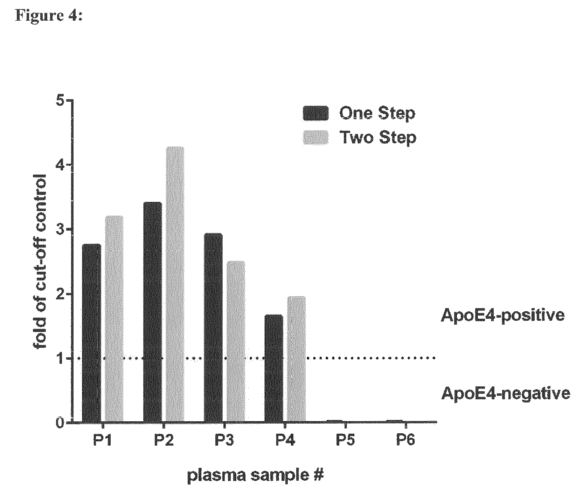

Here, a short (1 h) one-step procedure is compared to a long (overnight/ 2 h) two-step procedure. The relative light units (RLU) of the one-step assay are about ten times lower than the RLUs of the two-step assay (see table 2). But this ratio is true for every ApoE4 positive sample and the cut-off control. The values for ApoE4-negative samples stay equally low in any assay format. The sample values in relation to the internal cut-off control are about the same in every assay (see figure 4) and each assay is capable of differentiating ApoE4-positive from ApoE4-negative samples.

**Table 2:**

| **ApoE4 status** | **samples** | **One**-**Step** | | **Two**-**Step** | |
|---|---|---|---|---|---|
| | | **RLU** | **fold cut-off control** | **RLU** | **fold cut-off control** |
| positive | P1 | 16.126 | 2,75 | 162.422 | 3,19 |
| | P2 | 19.917 | 3,40 | 217.180 | 4,26 |
| | P3 | 17.034 | 2,91 | 126.767 | 2,49 |
| | P4 | 9.665 | 1,65 | 98.857 | 1,94 |
| negative | P5 | 79 | 0,0135 | 88 | 0,0017 |
| | P6 | 71 | 0,0121 | 62 | 0,0012 |
| Cut Off control | | 5.861 | 1,00 | 50.986 | 1,00 |

### 6. Example 6

Effect of blocking/ stabilizing agent (BSA) on ApoE4 binding to solid phase.

### 6.1 Methods

The ApoE4 assay was performed in a two-step procedure, where in the first step (ApoE4 binding to the solid phase) is performed in the presence or absence of BSA. The second step (binding of anti-ApoE4 antibody to immobilized ApoE4) is performed in assay buffer containing 1 % BSA.

Plasma samples and a low ApoE4-cut off control (recombinant ApoE4 [4 µg/ ml] in ApoE4-negative human plasma) were directly pipetted into wells of a 96-well microtiter plate (Greiner, Lumitrac600 high-binding, polystyrene; 20 µl sample per well). Wells were filled with 200 µl of reaction buffer (PBS with 0.2 % Tween-20, 0.1 % bovine IgGs, 0.1 % sheep IgGs and 0.02 % mouse IgGs, pH 7.4) that either contained 1 % BSA or did not contain BSA (0 % BSA). The microplates were incubated for 1 hour at room temperature (= step 1). Afterwards, microplates were washed 4 times with washing solution (20 mM PBS, pH 7.4, 0.1 % Triton X-100) and 200 µl of 1 % BSA containing reaction buffer with acridiniumester-labelled antiApoE4-antibody were filled into the wells. The microplates were incubated for 1 hour at room temperature (= step 2), and washed again 4 times with washing solution. Luminescence (in relative light units (RLU)) was measured using a MTP luminometer (Centro LB960, Berthold Technologies GmbH, Bad Wildbad).

In order to compare the two assay variants more directly, the RLU values were transformed into the relative units "folds of cut-off control".

### 6.2 Results

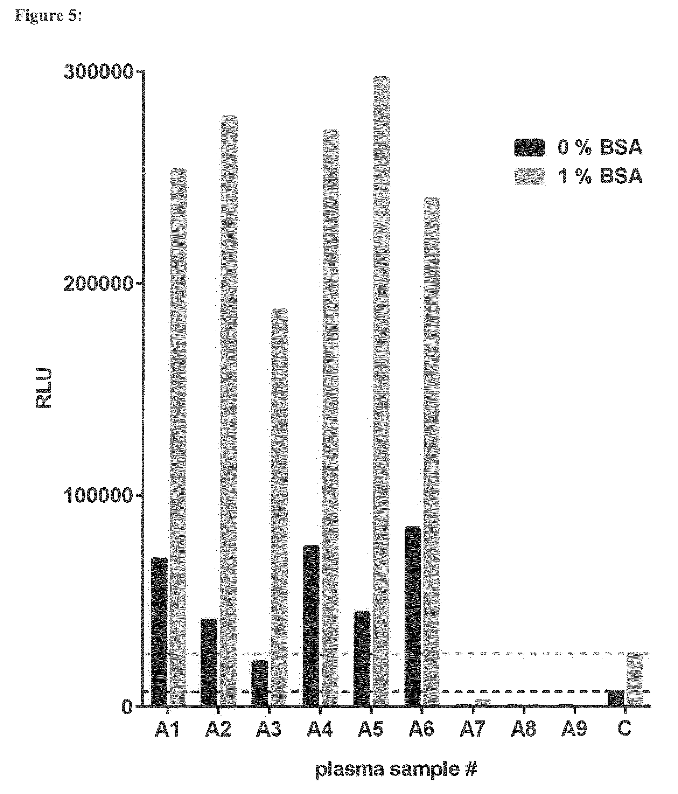

The signals of the samples incubated in buffer without BSA are significantly lower than, with BSA in the reaction mixture (see table 3, figure 5). Both assay variants are able to differentiate between ApoE4 positive and ApoE4 negative samples using the cut-off control. Consequently, BSA is not essential for ApoE4 binding, but surprisingly it strongly enhances ApoE4 binding to the solid phase and can thus be optionally added to the reaction buffer to increase signals. This result is surprising since BSA is usually used to block unspecific signals and is often accompanied by lower signals after the addition of BSA.

**Table 3:**

| **ApoE4 status** | **samples** | **0 % BSA** | | **1 % BSA** | |
|---|---|---|---|---|---|
| | | **RLU** | **fold cut-off control** | **RLU** | **fold cut-off control** |
| positive | A1 | 69.708 | 9,76 | 253.321 | 10,15 |
| | A2 | 40.772 | 5,71 | 278.431 | 11,16 |
| | A3 | 20.839 | 2,92 | 187.286 | 7,51 |
| | A4 | 75.446 | 10,57 | 271.817 | 10,89 |
| | A5 | 44.542 | 6,239 | 297.033 | 11,906 |
| | A6 | 84.069 | 11,776 | 239.914 | 9,616 |
| negative | A7 | 472 | 0,066 | 2.776 | 0,111 |
| | A8 | 401 | 0,056 | 244 | 0,010 |
| | A9 | 119 | 0,017 | 97 | 0,004 |
| Cut-off control | | 7.139 | 1 | 24.949 | 1 |

### 7. Example 7

Quantification of ApoE4 in plasma samples.

### 7.1 Methods

The ApoE4 Assay was performed as described in example 2. In order to quantify the ApoE4 levels in plasma, calibrators were used. Recombinant ApoE4 was diluted in ApoE4-negative human plasma in a stepwise manner, generating a calibration curve in the range of 0.01 - 21.87 µg/ ml. Sample ApoE4 concentration was calculated using the linear regression of the calibration curve. Samples that showed RLU values above the highest standard value were diluted in ApoE4-negative human plasma and remeasured. The cut-off control with the defined concentration of 4 µg/ ml is also measured, as control for the standard curve.

### 7.2 Results

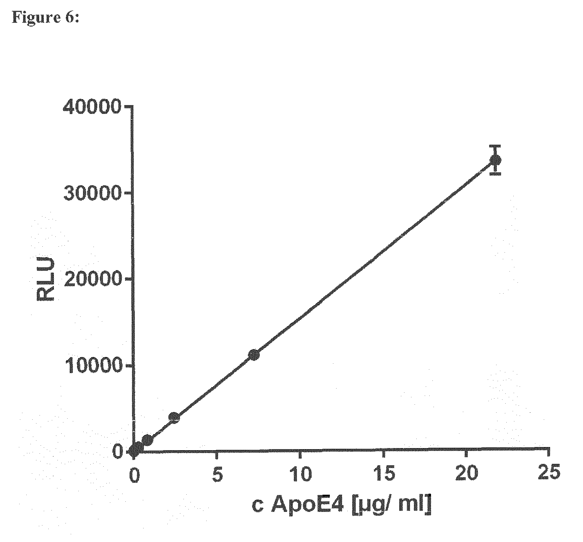

In the range of 0.01 - 21.87 µg/ ml ApoE4 is linear diluted (see figure 6). Table 4 shows the measured RLU values and calculated ApoE4 concentrations. ApoE4-positive samples have concentrations in the range of 9.7 - 30.4 µg/ ml. The concentration of all ApoE4 negative samples is always <0.1 µg/ ml (assay background). The concentration of the cut-off control is with 4 µg/ ml exactly the same measured with the calibrators as diluted before the measurement.

**Table 4:**

| **ApoE4 status** | **Samples** | **c ApoE4 [µg/ ml]** |
|---|---|---|
| positive | A | 22.3 |
| | B | 25.2 |
| | C | 9.7 |
| | D | 21.9 |
| | E | 30.4 |
| negative | F | <0.1 |
| | G | <0.1 |
| | H | <0.1 |
| cut-off control | | 4.0 |

### REFERENCES

Aggarwal NT, Wilson RS, Beck TL, et al. The apolipoprotein E epsilon4 allele and incident Alzheimer's disease in persons with mild cognitive impairment. Neurocase. 2005; 11(1):3-7.
Bakbak, B. et al., 2015. Association of Apolipoprotein E Polymorphism with Intravitreal Ranibizumab Treatment Outcomes in Age-Related Macular Degeneration. Current eye research, 3683(October), pp.1-5.
Bury, J. et al., 1986. Apolipoprotein E quantified by enzyme-linked immunosorbent assay. Clin Chem., 32(2), pp.265-70.
Cacabelos, R. et al., 2012. Genomics of dementia: APOE - And CYP2D6-related pharmacogenetics. International Journal of Alzheimer's Disease, 2012(518901).
Calero, O. et al., 2009. Apolipoprotein E genotyping method by Real Time PCR, a fast and cost-effective alternative to the TaqMan and FRET® assays. J Neurosci Methods, 183(2), pp.238-40.
Deng, B. et al., 2014. Aptamer binding assays for proteins: The thrombin example-A review. Analytica Chimica Acta, 837, pp.1-15.
Hegemann, D., Brunner, H. & Oehr, C., 2003. Plasma treatment of polymers for surface and adhesion improvement. Nuclear Instruments and Methods in Physics, 208, pp.281-286.
Hesse, C. et al., 2000. Measurement of Apolipoprotein E (apoE) in Cerebrospinal Fluid. Neurochemical Research, 25(4), pp.511-517.
Høgåsen, K. et al., 1993. Quantitation of vitronectin and clusterin Pitfalls and solutions in enzyme immunoassays for adhesive proteins. Journal of Immunological Methods, 160, pp.107-115.
Holmquist, L., 1982. Loss of human serum apolipoproteins C and E during manipulation of diluted solutions. Journal of Lipid Research, 23, pp.357-359.
Kirk-Othmer, Encyclopedia of chemical technology, 4th ed., executive editor, J. I. Kroschwitz; editor, M. Howe-Grant, John Wiley & Sons, 1993, vol.15, p. 518-562, incorporated herein by reference, including citations on pages 551-562
Lane, R.D. (1985). A short-duration polyethylene glycol fusiontechnique for increasing production of monoclonal antibody-secreting hybridomas. J. Immunol. Meth. 81: 223-228
Leduc, V. et al., 2011. Function and comorbidities of apolipoprotein e in Alzheimer's disease. International journal of Alzheimer's disease, 2011(974361).
Li, J. et al., 2011. Peptide aptamers with biological and therapeutic applications. Current medicinal chemistry, 18(27), pp.4215-22.
Love, J.E. et al. 2015. Alternative splicing in Alzheimer's Disease. J Parkinsons Dis Alzheimers Dis. 2(2). pii: 6.
Marx et al., Monoclonal Antibody Production, ATLA 25, 121, 1997
Mayeux, R. et al., 1998. Utility of the Apolipoprteoin E Genotype in the Diagnosis of Alzheimer's Disease. N Engl J Med, 338(8), pp.506-511.
Rohn, T.T. 2013. Proteolytic cleavage of apolipoprotein E4 as the keystone for the heightened risk associated with Alzheimer's disease, Int J Mol Sci. 14(7): 14908-22.
Sanger F; Coulson AR (May 1975). "A rapid method for determining sequences in DNA by primed synthesis with DNA polymerase". J. Mol. Biol. 94 (3): 441-8
Verghese, P.B., Castellano, J.M. & Holtzman, D.M., 2011. Roles of Apolipoprotein E in Alzheimer's disease and other neurological disorders. Lancet neurology, 10(3), pp.241-252.
Weisgraber, K.H. & Mahley, R.W., 1996. Human apolipoprotein E: the Alzheimer's disease connection. Federation of American Societies for Experimental Biology, 10, pp.1485-94.
Xu, Y., Yang, X. & Wang, E., 2010. Review: Aptamers in microfluidic chips. Analytica chimica acta, 683(1), pp. 12-20.
Ziegler, B. et al. (1996) Glutamate decarboxylase (GAD) is not detectable on the surface of rat islet cells examined by cytofluorometry and complement-dependent antibody-mediated cytotoxicity of monoclonal GAD antibodies, Horm. Metab. Res. 28: 11-15)
Zhong, L. et al., 2016. A rapid and cost-effective method for genotyping apolipoprotein E gene polymorphism. Molecular Neurodegeneration, 11(2), pp. 1-8.

### SEQUENCES

**SEQ ID NO: 1** 
10 20 30 40 50
KVEQAVETEP EPELRQQTEW QSGQRWELAL GRFWDYLRWV QTLSEQVQEE
60 70 80 90 100
LLSSQVTQEL RALMDETMKE LKAYKSELEE QLTPVAEETR ARLSKELQAA
110 120 130 140 150
QARLGADMED VRGRLVQYRG EVQAMLGQST EELRVRLASH LRKLRKRLLR
160 170 180 190 200
DADDLQKRLA VYQAGAREGA ERGLSAIRER LGPLVEQGRV RAATVGSLAG
210 220 230 240 250
QPLQERAQAW GERLRARMEE MGSRTRDRLD EVKEQVAEVR AKLEEQAQQI
260 270 280 290
RLQAEAFQAR LKSWFEPLVE DMQRQWAGLV EKVQAAVGTS AAPVPSDNH
**SEQ ID NO: 2 (human preApoE4 1-317, including signal sequence)** 
10 20 30 40 50

60 70 80 90 100

110 120 130 140 150

160 170 180 190 200

210 220 230 240 250

260 270 280 290 300

310

**SEQ ID NO.: 3 (Apo E4 fragment 1-165)** 
KVEQAVETEP EPELRQQTEW QSGQRWELAL GRFWDYLRWV QTLSEQVQEE
LLSSQVTQEL RALMDETMKE LKAYKSELEE QLTPVAEETR ARLSKELQAA
QARLGADMED VRGRLVQYRG EVQAMLGQST EELRVRLASH LRKLRKRLLR
DADDLQKRLA VYQAG
**SEQ ID NO.: 4** (**ApoE4 fragment 1**-**185)** 
KVEQAVETEP EPELRQQTEW QSGQRWELAL GRFWDYLRWV QTLSEQVQEE
LLSSQVTQEL RALMDETMKE LKAYKSELEE QLTPVAEETR ARLSKELQAA
QARLGADMED VRGRLVQYRG EVQAMLGQST EELRVRLASH LRKLRKRLLR
DADDLQKRLA VYQAGAREGA ERGLSAIRER LGPLV
**SEQ ID NO.: 5** (**ApoE4 fragment 1-191)** 
KVEQAVETEP EPELRQQTEW QSGQRWELAL GRFWDYLRWV QTLSEQVQEE
LLSSQVTQEL RALMDETMKE LKAYKSELEE QLTPVAEETR ARLSKELQAA
QARLGADMED VRGRLVQYRG EVQAMLGQST EELRVRLASH LRKLRKRLLR
DADDLQKRLA VYQAGAREGA ERGLSAIRER LGPLVEQGRV R
**SEQ ID NO.: 6 (ApoE4 fragment 1-202)** 
KVEQAVETEP EPELRQQTEW QSGQRWELAL GRFWDYLRWV QTLSEQVQEE

LLSSQVTQEL RALMDETMKE LKAYKSELEE QLTPVAEETR ARLSKELQAA

QARLGADMED VRGRLVQYRG EVQAMLGQST EELRVRLASH LRKLRKRLLR

DADDLQKRLA VYQAGAREGA ERGLSAIRER LGPLVEQGRV RAATVGSLAG

QP
**SEQ ID NO.: 7** (**ApoE4 fragment 1-229)** 
KVEQAVETEP EPELRQQTEW QSGQRWELAL GRFWDYLRWV QTLSEQVQEE

LLSSQVTQEL RALMDETMKE LKAYKSELEE QLTPVAEETR ARLSKELQAA

QARLGADMED VRGRLVQYRG EVQAMLGQST EELRVRLASH LRKLRKRLLR

DADDLQKRLA VYQAGAREGA ERGLSAIRER LGPLVEQGRV RAATVGSLAG

QPLQERAQAW GERLRARMEE MGSRTRDRL
**SEQ ID NO.: 8** (**ApoE4 fragment 1-231)** KVEQAVETEP EPELRQQTEW QSGQRWELAL GRFWDYLRWV QTLSEQVQEE 
LLSSQVTQEL RALMDETMKE LKAYKSELEE QLTPVAEETR ARLSKELQAA
QARLGADMED VRGRLVQYRG EVQAMLGQST EELRVRLASH LRKLRKRLLR
DADDLQKRLA VYQAGAREGA ERGLSAIRER LGPLVEQGRV RAATVGSLAG
QPLQERAQAW GERLRARMEE MGSRTRDRLD E
**SEQ ID NO.: 9 (fragment ApoE4 1-251)** 
KVEQAVETEP EPELRQQTEW QSGQRWELAL GRFWDYLRWV QTLSEQVQEE
LLSSQVTQEL RALMDETMKE LKAYKSELEE QLTPVAEETR ARLSKELQAA
QARLGADMED VRGRLVQYRG EVQAMLGQST EELRVRLASH LRKLRKRLLR
DADDLQKRLA VYQAGAREGA ERGLSAIRER LGPLVEQGRV RAATVGSLAG
QPLQERAQAW GERLRARMEE MGSRTRDRLD EVKEQVAEVR AKLEEQAQQI
R
**SEQ ID NO.: 10 (fragment ApoE4 1-259)** 
KVEQAVETEP EPELRQQTEW QSGQRWELAL GRFWDYLRWV QTLSEQVQEE
LLSSQVTQEL RALMDETMKE LKAYKSELEE QLTPVAEETR ARLSKELQAA
QARLGADMED VRGRLVQYRG EVQAMLGQST EELRVRLASH LRKLRKRLLR
DADDLQKRLA VYQAGAREGA ERGLSAIRER LGPLVEQGRV RAATVGSLAG
QPLQERAQAW GERLRARMEE MGSRTRDRLD EVKEQVAEVR AKLEEQAQQI
RLQAEAFQA
**SEQ ID NO.: 11 (fragment ApoE4 1-260)** 
KVEQAVETEP EPELRQQTEW QSGQRWELAL GRFWDYLRWV QTLSEQVQEE
LLSSQVTQEL RALMDETMKE LKAYKSELEE QLTPVAEETR ARLSKELQAA
QARLGADMED VRGRLVQYRG EVQAMLGQST EELRVRLASH LRKLRKRLLR
DADDLQKRLA VYQAGAREGA ERGLSAIRER LGPLVEQGRV RAATVGSLAG
QPLQERAQAW GERLRARMEE MGSRTRDRLD EVKEQVAEVR AKLEEQAQQI
RLQAEAFQAR
**SEQ ID NO.: 12 (fragment ApoE4 1-266)** 
KVEQAVETEP EPELRQQTEW QSGQRWELAL GRFWDYLRWV QTLSEQVQEE
LLSSQVTQEL RALMDETMKE LKAYKSELEE QLTPVAEETR ARLSKELQAA
QARLGADMED VRGRLVQYRG EVQAMLGQST EELRVRLASH LRKLRKRLLR
DADDLQKRLA VYQAGAREGA ERGLSAIRER LGPLVEQGRV RAATVGSLAG
 
QPLQERAQAW GERLRARMEE MGSRTRDRLD EVKEQVAEVR AKLEEQAQQI
RLQAEAFQAR LKSWFE
**SEQ ID NO.: 13 (fragment ApoE4 1-271)** 
KVEQAVETEP EPELRQQTEW QSGQRWELAL GRFWDYLRWV QTLSEQVQEE
LLSSQVTQEL RALMDETMKE LKAYKSELEE QLTPVAEETR ARLSKELQAA
QARLGADMED VRGRLVQYRG EVQAMLGQST EELRVRLASH LRKLRKRLLR
DADDLQKRLA VYQAGAREGA ERGLSAIRER LGPLVEQGRV RAATVGSLAG
QPLQERAQAW GERLRARMEE MGSRTRDRLD EVKEQVAEVR AKLEEQAQQI
RLQAEAFQAR LKSWFEPLVE D
**SEQ ID NO.: 14 (fragment ApoE4 1-272)** 
KVEQAVETEP EPELRQQTEW QSGQRWELAL GRFWDYLRWV QTLSEQVQEE
LLSSQVTQEL RALMDETMKE LKAYKSELEE QLTPVAEETR ARLSKELQAA
QARLGADMED VRGRLVQYRG EVQAMLGQST EELRVRLASH LRKLRKRLLR
DADDLQKRLA VYQAGAREGA ERGLSAIRER LGPLVEQGRV RAATVGSLAG
QPLQERAQAW GERLRARMEE MGSRTRDRLD EVKEQVAEVR AKLEEQAQQI
RLQAEAFQAR LKSWFEPLVE DM
**SEQ ID NO.: 15 (ApoE4 72-299)**

KAYKSELEEQ LTPVAEETRA RLSKELQAAQ ARLGADMEDV RGRLVQYRGE
VQAMLGQSTE ELRVRLASHL RKLRKRLLRD ADDLQKRLAV YQAGAREGAE
RGLSAIRERL GPLVEQGRVR AATVGSLAGQ PLQERAQAWG ERLRARMEEM
GSRTRDRLDE VKEQVAEVRA KLEEQAQQIR LQAEAFQARL KSWFEPLVED
MQRQWAGLVE KVQAAVGTSA APVPSDNH

### FIGURE DESCRIPTIONS

**Figure 1****:**
   (A) Measurement of plasma samples with known ApoE genotype. Plasma ApoE4 concentrations correlate with genetic status (low values for ApoE4 negative samples, high values for ApoE4 positive samples; statistical analysis: Mann-Whitney test).
   (B) ROC analysis of the ApoE4 assay with the genotype (AUC - area under the curve).
**Figure 2****:**
   Measurement of ApoE4 levels after pre-incubation in tubes of different material with and without the addition of detergent (PS - polystyrene, PP - polypropylene, PET - polyethylene terephthalate).
**Figure 3****:**
   Measurement of ApoE4 levels in MTPs after pre-treatment of MTPs with/ without detergent (Triton X-100). Assay buffer contained Triton X-100 (0.05 %) or did not contain any detergent (0 %), samples were measured in a one-step procedure. The cut-off control (A), the two ApoE4 positive samples D1 (B) and D2 (C) and the ApoE4 negative sample D3 (D) are shown here.
**Figure 4**
   Comparison of one-step and two-step ApoE4 assay. The dotted line shows the value of the cut off control. Every sample with values below such a cut-off is ApoE4 negative and every sample with values above the threshold is ApoE4 positive.
**Figure 5****:**
   Analysis of ApoE4 binding to solid phase in the presence (1 %)/ absence (0 %) of BSA. The dotted line shows the value of the cut-off control (C) in the respective assay variant.
**Figure 6****:**
   Linear regression of ApoE4-standards in the range of 0.01 and 21.87 µg/ ml.

## Claims

1. *(In vitro)* Method for the detection of Apolipoprotein E isotype 4 (ApoE4) or fragments thereof in a blood sample of a subject comprising the steps of:
a. contacting said sample with a solid phase,
contacting said sample simultaneously or sequentially with at least one binder binding specifically to ApoE4, thereby forming an ApoE4-binder-complex, wherein said binder is an antibody or a functional antibody fragment or non-IgG protein scaffold, and
b. detecting the ApoE4-binder-complex, wherein said ApoE4-binder-complex is immobilized to said solid phase by means of ApoE4.

2. The method according to claim 1, wherein ApoE4 or fragments thereof is immobilized to said solid phase and the binder is bound to ApoE4.

3. The method according to claims 1 to 2, wherein the fragments of ApoE4 comprise at least 6 amino acids in length with the proviso that the amino acid 158 (Arginine) is encompassed within said fragment, whereby the sequence of the amino acid 158 refers to Seq ID NO.1.

4. The method according to claims 1 to 3, wherein the binding of ApoE4 or the ApoE4-binder-complex to said solid phase occurs without the interaction of a connecting molecule, which is able to bind ApoE4 or fragments thereof to said solid phase.

5. The method according to claims 1 to 4, wherein at the moment of contacting the solid phase by the sample, said contacting occurs without the interaction of said connecting molecule.

6. The method according to claims 1 to 5, wherein binding of ApoE4 or the ApoE4-binder-complex to said solid phase occurs in the presence of a detergent.

7. The method according to claims 1 to 6, wherein the ApoE4-binder-complex is detected qualitatively or quantitatively.

8. The method according to claims 1 to 7, wherein a quantitative detection of the ApoE4-binder-complex indicates the concentration of ApoE4 in said sample, wherein the concentration provides an indication whether the subjects are homozygous or heterozygous for ApoE4 alleles.

9. The method according to claims 1 to 8, wherein the binder is an antibody or a functional antibody fragment or non-IgG protein scaffold with a binding affinity constant of at least 10⁷ M⁻¹, preferred 10⁸ M⁻¹, preferred affinity constant is greater than 10⁹ M⁻¹, most preferred greater than 10¹⁰ M⁻¹.

10. The method according to claims 1 to 9, wherein the binder is labelled with a label in order to be detected, wherein said label is seleeted from the group comprising chemiluminescent label, enzyme label, fluorescence label or radioiodine label.

11. The method according to claims 1 to 10, wherein the detection of ApoE4 is used for a classification of a subject to be ApoE4 positive or ApoE4 negative, when the level of the detected ApoE4 is above a certain threshold, wherein said threshold is determined by a specific internal positive control.

12. The method according to claims 1 to 11, wherein the solid phase is selected from the group of polymeric surface (e.g. polystyrene), glass surface, cellulose and cellulose derivatives (e.g. nitrocellulose, polyvinylidene fluoride, nylon).

13. The method according to claims 1 to 12, wherein the solid phase is selected from the group of glass or polymeric surface, wherein polymeric surface comprises but is not limited to polystyrene, poly(divinylbenzene), styrene-acylate copolymer, styrenebutadiene copolymer, styrene-divinylbenzene copolymer, poly(styrene-oxyethylene), polymethyl methacrylate, polyurethane, polyglutaraldehyde, polyethylene imine, polyvinylpyrrolidone, N,N'-methylene bis-acrylamide, polyolefeins, polyethylene, polytetrafluoroethylene, polyethylene terephthalate, polypropylene, polyvinylchloride, polyvinylacetate, polyacrylonitrile, polysulfone, poly(ether sulfone), polydimethylsiloxane, pyrolized materials, block copolymers, and copolymers of the foregoing, silicones, or silica.

14. The method according to claims 1 to 13, wherein said blood sample is contacted with an unsaturated solid phase.

15. The method according to claims 1 to 14, wherein the solid face is not covered with any binder or capture molecule.

16. The method according to claims 1 to 15, wherein an optional stabilizing protein in the reaction buffer stabilizes the binding of ApoE4 and fragments thereof in the blood sample to the solid phase by blocking unspecific binding sites and interacting with the conformation of ApoE4, wherein the stabilizing protein is selected from the group comprising bovine serum albumin (BSA), albumin from human serum, casein, milk proteins, immunoglobulins and stabilizing amino acids (e.g. Alanine, Arginine, Glycine, Isoleucine, Lysine, Proline, Serine), preferably the stabilizing protein is bovine serum albumin (BSA).

17. The method according to claims 1-16, wherein the concentration of BSA as stabilizing protein is in the range of 0-10 %, preferably 0.2-5%, most preferably 0.5-2.5 %.

18. The method according to claims 1-17, wherein the detergent is selected from the group of anionic detergents (e.g. sodium dodecylsulfate (SDS)), cationic detergents (e.g. Cetyltrimethylammonium bromide (CTAB)), zwitterionic detergents (e.g. CHAPS) and/ or non-ionic detergents (e.g. Tween-20, Triton X-100).

19. The method according to claims 1-18, wherein the Tween-20 concentration is selected from a range of 0.001-10%, preferably 0.01-1 %, most preferably 0.2-0.5 %.

20. The method according to claims 1-19, wherein the Triton X-100 concentration is selected from a range of 0.001-0.5 %, preferably 0.01-0.2 %, most preferably 0.05-0.1 %.

21. The method according to any of the preceding claims, wherein the blood sample is selected from the group of whole blood, serum and citrate plasma, heparin plasma, EDTA-plasma.

22. The method according to any of the preceding claims, wherein the subject is selected from the group of living human or human-organism, preferably a human subject, wherein the subject is healthy, apparently healthy, diseased with cognitive impairment dementia, in particular AD.

23. A Kit comprising
a. one binder directed against an epitope comprised in the sequence of ApoE4, wherein the binder is labelled with a detectable label,
b. a solid phase,
c. a detergent containing reaction buffer.

24. Use of a kit according to any of the preceding claims for the *in vitro* detection and/or quantification of ApoE4 in a blood sample of a subject.

25. Use of the method according to any of the preceding claims for the risk stratification, or stratification for application of therapeutic measures of a disease or condition associated with elevated ApoE4 levels.

26. Use of the method according to any of the preceding claims, wherein the disease or condition is selected from the group of cognitive impairment and neurodegenerative diseases including dementia and/or Alzheimer's disease (AD).

27. A method according to any of the preceding claims for detecting the risk to develop AD, wherein an elevated level of ApoE4 above a certain threshold is predictive for an enhanced risk of developing AD.

28. The method according to any of the preceding claims, wherein said presence of ApoE4 or the ApoE4-binder-complex is correlated with a risk of developing/incidence of AD.


SEQUENCE LISTING

<110>  sphingotec GmbH

<120>  METHOD FOR THE DETECTION OF APOLIPOPROTEIN E4

<130>  S75097EP

<160>  15

<170>  PatentIn version 3.5

<210>  1
<211>  299
<212>  PRT
<213>  Homo sapiens

<400>  1

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Arg
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val Glu Gln Gly Arg Val Arg Ala
            180                 185                 190

Ala Thr Val Gly Ser Leu Ala Gly Gln Pro Leu Gln Glu Arg Ala Gln
        195                 200                 205

Ala Trp Gly Glu Arg Leu Arg Ala Arg Met Glu Glu Met Gly Ser Arg
    210                 215                 220

Thr Arg Asp Arg Leu Asp Glu Val Lys Glu Gln Val Ala Glu Val Arg
225                 230                 235                 240

Ala Lys Leu Glu Glu Gln Ala Gln Gln Ile Arg Leu Gln Ala Glu Ala
                245                 250                 255

Phe Gln Ala Arg Leu Lys Ser Trp Phe Glu Pro Leu Val Glu Asp Met
            260                 265                 270

Gln Arg Gln Trp Ala Gly Leu Val Glu Lys Val Gln Ala Ala Val Gly
        275                 280                 285

Thr Ser Ala Ala Pro Val Pro Ser Asp Asn His
    290                 295

<210>  2
<211>  317
<212>  PRT
<213>  Homo sapiens

<400>  2

<210>  3
<211>  165
<212>  PRT
<213>  Homo sapiens

<400>  3

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Arg
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly
                165

<210>  4
<211>  185
<212>  PRT
<213>  Homo sapiens

<400>  4

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Arg
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val
            180                 185

<210>  5
<211>  191
<212>  PRT
<213>  Homo sapiens

<400>  5

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Arg
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val Glu Gln Gly Arg Val Arg
            180                 185                 190

<210>  6
<211>  202
<212>  PRT
<213>  Homo sapiens

<400>  6

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Arg
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val Glu Gln Gly Arg Val Arg Ala
            180                 185                 190

Ala Thr Val Gly Ser Leu Ala Gly Gln Pro
        195                 200

<210>  7
<211>  229
<212>  PRT
<213>  Homo sapiens

<400>  7

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Arg
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val Glu Gln Gly Arg Val Arg Ala
            180                 185                 190

Ala Thr Val Gly Ser Leu Ala Gly Gln Pro Leu Gln Glu Arg Ala Gln
        195                 200                 205

Ala Trp Gly Glu Arg Leu Arg Ala Arg Met Glu Glu Met Gly Ser Arg
    210                 215                 220

Thr Arg Asp Arg Leu
225

<210>  8
<211>  231
<212>  PRT
<213>  Homo sapiens

<400>  8

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Arg
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val Glu Gln Gly Arg Val Arg Ala
            180                 185                 190

Ala Thr Val Gly Ser Leu Ala Gly Gln Pro Leu Gln Glu Arg Ala Gln
        195                 200                 205

Ala Trp Gly Glu Arg Leu Arg Ala Arg Met Glu Glu Met Gly Ser Arg
    210                 215                 220

Thr Arg Asp Arg Leu Asp Glu
225                 230

<210>  9
<211>  251
<212>  PRT
<213>  Homo sapiens

<400>  9

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Arg
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val Glu Gln Gly Arg Val Arg Ala
            180                 185                 190

Ala Thr Val Gly Ser Leu Ala Gly Gln Pro Leu Gln Glu Arg Ala Gln
        195                 200                 205

Ala Trp Gly Glu Arg Leu Arg Ala Arg Met Glu Glu Met Gly Ser Arg
    210                 215                 220

Thr Arg Asp Arg Leu Asp Glu Val Lys Glu Gln Val Ala Glu Val Arg
225                 230                 235                 240

Ala Lys Leu Glu Glu Gln Ala Gln Gln Ile Arg
                245                 250

<210> 10
<211> 259
<212> PRT
<213> Homo sapiens

<400> 10

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Arg
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val Glu Gln Gly Arg Val Arg Ala
            180                 185                 190

Ala Thr Val Gly Ser Leu Ala Gly Gln Pro Leu Gln Glu Arg Ala Gln
        195                 200                 205

Ala Trp Gly Glu Arg Leu Arg Ala Arg Met Glu Glu Met Gly Ser Arg
    210                 215                 220

Thr Arg Asp Arg Leu Asp Glu Val Lys Glu Gln Val Ala Glu Val Arg
225                 230                 235                 240

Ala Lys Leu Glu Glu Gln Ala Gln Gln Ile Arg Leu Gln Ala Glu Ala
                245                 250                 255

Phe Gln Ala

<210>  11
<211>  260
<212>  PRT
<213>  Homo sapiens

<400>  11

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Arg
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val Glu Gln Gly Arg Val Arg Ala
            180                 185                 190

Ala Thr Val Gly Ser Leu Ala Gly Gln Pro Leu Gln Glu Arg Ala Gln
        195                 200                 205

Ala Trp Gly Glu Arg Leu Arg Ala Arg Met Glu Glu Met Gly Ser Arg
    210                 215                 220

Thr Arg Asp Arg Leu Asp Glu Val Lys Glu Gln Val Ala Glu Val Arg
225                 230                 235                 240

Ala Lys Leu Glu Glu Gln Ala Gln Gln Ile Arg Leu Gln Ala Glu Ala
                245                 250                 255

Phe Gln Ala Arg
            260

<210>  12
<211>  266
<212>  PRT
<213>  Homo sapiens

<400>  12

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Arg
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val Glu Gln Gly Arg Val Arg Ala
            180                 185                 190

Ala Thr Val Gly Ser Leu Ala Gly Gln Pro Leu Gln Glu Arg Ala Gln
        195                 200                 205

Ala Trp Gly Glu Arg Leu Arg Ala Arg Met Glu Glu Met Gly Ser Arg
    210                 215                 220

Thr Arg Asp Arg Leu Asp Glu Val Lys Glu Gln Val Ala Glu Val Arg
225                 230                 235                 240

Ala Lys Leu Glu Glu Gln Ala Gln Gln Ile Arg Leu Gln Ala Glu Ala
                245                 250                 255

Phe Gln Ala Arg Leu Lys Ser Trp Phe Glu
            260                 265

<210>  13
<211>  271
<212>  PRT
<213>  Homo sapiens

<400>  13

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
            20                  25                  30

Phe Trp Asp Tyr Leu Arg Trp Val Gln Thr Leu Ser Glu Gln Val Gln
        35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Arg
            100                 105                 110

Gly Arg Leu Val Gln Tyr Arg Gly Glu Val Gln Ala Met Leu Gly Gln
        115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val Glu Gln Gly Arg Val Arg Ala
                180                 185                 190

Ala Thr Val Gly Ser Leu Ala Gly Gln Pro Leu Gln Glu Arg Ala Gln
            195                 200                 205

Ala Trp Gly Glu Arg Leu Arg Ala Arg Met Glu Glu Met Gly Ser Arg
    210                 215                 220

Thr Arg Asp Arg Leu Asp Glu Val Lys Glu Gln Val Ala Glu Val Arg
225                 230                 235                 240

Ala Lys Leu Glu Glu Gln Ala Gln Gln Ile Arg Leu Gln Ala Glu Ala
                245                 250                 255

Phe Gln Ala Arg Leu Lys Ser Trp Phe Glu Pro Leu Val Glu Asp
                260                 265                 270

<210>  14
<211>  272
<212>  PRT
<213>  Homo sapiens

<400>  14

Lys Val Glu Gln Ala Val Glu Thr Glu Pro Glu Pro Glu Leu Arg Gln
1               5                   10                  15

Gln Thr Glu Trp Gln Ser Gly Gln Arg Trp Glu Leu Ala Leu Gly Arg
                20                  25                  30

Phe Trp Asp Tyr Leu Arg Trp Val Gln Thr Leu Ser Glu Gln Val Gln
            35                  40                  45

Glu Glu Leu Leu Ser Ser Gln Val Thr Gln Glu Leu Arg Ala Leu Met
    50                  55                  60

Asp Glu Thr Met Lys Glu Leu Lys Ala Tyr Lys Ser Glu Leu Glu Glu
65                  70                  75                  80

Gln Leu Thr Pro Val Ala Glu Glu Thr Arg Ala Arg Leu Ser Lys Glu
                85                  90                  95

Leu Gln Ala Ala Gln Ala Arg Leu Gly Ala Asp Met Glu Asp Val Arg
                100                 105                 110

Gly Arg Leu Val Gln Tyr Arg Gly Glu Val Gln Ala Met Leu Gly Gln
            115                 120                 125

Ser Thr Glu Glu Leu Arg Val Arg Leu Ala Ser His Leu Arg Lys Leu
    130                 135                 140

Arg Lys Arg Leu Leu Arg Asp Ala Asp Asp Leu Gln Lys Arg Leu Ala
145                 150                 155                 160

Val Tyr Gln Ala Gly Ala Arg Glu Gly Ala Glu Arg Gly Leu Ser Ala
                165                 170                 175

Ile Arg Glu Arg Leu Gly Pro Leu Val Glu Gln Gly Arg Val Arg Ala
            180                 185                 190

Ala Thr Val Gly Ser Leu Ala Gly Gln Pro Leu Gln Glu Arg Ala Gln
        195                 200                 205

Ala Trp Gly Glu Arg Leu Arg Ala Arg Met Glu Glu Met Gly Ser Arg
    210                 215                 220

Thr Arg Asp Arg Leu Asp Glu Val Lys Glu Gln Val Ala Glu Val Arg
225                 230                 235                 240

Ala Lys Leu Glu Glu Gln Ala Gln Gln Ile Arg Leu Gln Ala Glu Ala
                245                 250                 255

Phe Gln Ala Arg Leu Lys Ser Trp Phe Glu Pro Leu Val Glu Asp Met
            260                 265                 270

<210>  15
<211>  228
<212>  PRT
<213>  Homo sapiens

<400>  15

Lys Ala Tyr Lys Ser Glu Leu Glu Glu Gln Leu Thr Pro Val Ala Glu
1               5                   10                  15

Glu Thr Arg Ala Arg Leu Ser Lys Glu Leu Gln Ala Ala Gln Ala Arg
            20                  25                  30

Leu Gly Ala Asp Met Glu Asp Val Arg Gly Arg Leu Val Gln Tyr Arg
        35                  40                  45

Gly Glu Val Gln Ala Met Leu Gly Gln Ser Thr Glu Glu Leu Arg Val
    50                  55                  60

Arg Leu Ala Ser His Leu Arg Lys Leu Arg Lys Arg Leu Leu Arg Asp
65                  70                  75                  80

Ala Asp Asp Leu Gln Lys Arg Leu Ala Val Tyr Gln Ala Gly Ala Arg
                85                  90                  95

Glu Gly Ala Glu Arg Gly Leu Ser Ala Ile Arg Glu Arg Leu Gly Pro
            100                 105                 110

Leu Val Glu Gln Gly Arg Val Arg Ala Ala Thr Val Gly Ser Leu Ala
        115                 120                 125

Gly Gln Pro Leu Gln Glu Arg Ala Gln Ala Trp Gly Glu Arg Leu Arg
    130                 135                 140

Ala Arg Met Glu Glu Met Gly Ser Arg Thr Arg Asp Arg Leu Asp Glu
145                 150                 155                 160

Val Lys Glu Gln Val Ala Glu Val Arg Ala Lys Leu Glu Glu Gln Ala
                165                 170                 175

Gln Gln Ile Arg Leu Gln Ala Glu Ala Phe Gln Ala Arg Leu Lys Ser
            180                 185                 190

Trp Phe Glu Pro Leu Val Glu Asp Met Gln Arg Gln Trp Ala Gly Leu
        195                 200                 205

Val Glu Lys Val Gln Ala Ala Val Gly Thr Ser Ala Ala Pro Val Pro
    210                 215                 220

Ser Asp Asn His
225